# EUROPÄISCHE PATENTSCHRIFT

(11) **EP 3 924 102 B1**
(45) Veröffentlichungstag und Bekanntmachung des Hinweises auf die Patenterteilung: **11.12.2024**
(21) Anmeldenummer: 19705954.6
(22) Anmeldetag: 13.02.2019
(51) Int. Cl.: B01J 13/14, A01N 25/28, A23P 10/30, A61K 8/11, A61K 9/50, B01J 13/20, B01J 13/22, C09B 67/02, C11D 3/50

(54) **VERFAHREN ZUR HERSTELLUNG VON MIKROKAPSELN**
METHOD FOR PRODUCING MICROCAPSULES
PROCÉDÉ DE PRODUCTION DE MICROCAPSULES

(43) Veröffentlichungstag der Anmeldung: 22.12.2021
(73) Patentinhaber: Symrise AG, 37603 Holzminden (DE)
(72) Erfinder: GEORGI, Julian Alexander, 37603 Holzminden (DE); ROST, Benjamin, 37619 Bodenwerder (DE); DRÖGE, Diane, 37619 Bodenwerder (DE); BERTRAM, Ralf, 37603 Holzminden (DE); BÖDDEKER, Thorsten, 33034 Brakel (DE)
(74) Vertreter: Global IP Europe Patentanwaltskanzlei
(86) Internationale Anmeldenummer: PCT/EP2019/053600
(87) Internationale Veröffentlichungsnummer: WO 2020/164705

(56) Entgegenhaltungen:
- EP-A2- 3 300 794
- WO-A1-2016/144798
- WO-A1-2017/058875
- US-A1- 2013 337 023
- US-A1- 2015 252 312
- US-A1- 2017 252 274

## Beschreibung

### Gebiet der Erfindung

Die vorliegende Erfindung betrifft ein Verfahren zur Herstellung von Isocyanat-basierten oder Isothiocyanat-basierten Mikrokapseln, mit einer verbesserten Stabilität gegenüber Carbonsäure, Aldehyd und/oder Ester enthaltenden Wirkstoffen als Kernmaterial. Darüber hinaus betrifft die vorliegende Erfindung Mikrokapseln, die nach dem erfindungsgemäßen Verfahren erhältlich sind. Außerdem betrifft die vorliegende Erfindung Mikrokapseln, Suspensionen solcher Mikrokapseln sowie die Verwendung der Mikrokapseln und Suspensionen als Bestandteil von Waschmitteln, Weichspülern, Reinigungsmitteln, Duftverstärkern in flüssiger oder fester Form, Kosmetika, Körperpflegeprodukten, Agrarprodukten und pharmazeutischen Produkten.

Unter dem Begriff "Verkapselung" versteht der Fachmann im Allgemeinen eine Technik, mit der feindisperse feste, flüssige oder gasförmige Substanzen mit einer filmbildenden Hülle aus polymeren oder anorganischen Wandmaterialien umgeben werden und so immobilisiert werden. Bei der Herstellung schlagen sich die Polymere nach Emulgierung und Koazervation oder Grenzflächenpolymerisation auf den einzuhüllenden Substanzen nieder. Die Verkapselung ist somit eine Form der Einschlussimmobilisierung. Die in den Kapseln eingeschlossenen Substanzen bzw. Wirkstoffe werden allgemein als Kernmaterial bezeichnet.

Ziel ist es dabei, die als Wirkstoffe bezeichneten Aktivkomponenten zu schützen und zu einem bestimmten Zeitpunkt gezielt freisetzen zu können. Die Wirkstoffe bzw. Aktivkomponenten können oft aus verschiedenen Gründen (beispielsweise aufgrund ihrer Löslichkeit, Reaktivität, Stabilität etc.) nicht direkt eingesetzt werden, oder es sollen bestimmte Wirkungen durch die Mikroverkapselung erzielt werden (z. B. Freisetzungskurven für Controlled Release, Alleinstellungsmerkmale etc.).

Bei einem Durchmesser im Bereich von > 100 µm werden einzelne Substanz- oder Wirkstoffpartikel mit verschiedenen Sprühtechnologien beschichtet, Flüssigkeiten hingegen werden über Vertropfungsverfahren eingekapselt.

Von besonderem Interesse sind sogenannte Mikrokapseln, die Durchmesser im Bereich von etwa 0,0001 bis 5, vorzugsweise 0,001 bis 0,5 und insbesondere 0,005 bis 0,1 mm aufweisen.

Für Mikrokapseln mit einem Durchmesser unter 100 µm gelingt eine Verkapselung jedoch nur durch Abscheiden sogenannter Wandbildner an einer Phasengrenzfläche. Im hierfür industriell genutzten Herstellungsverfahren wird eine Emulsion oder Suspension des hydrophoben Kernmaterials in Wasser erzeugt. Die in der äußeren Phase befindlichen Monomere lagern sich in einer Schicht um die Emulsionstropfen oder die dispergierten Feststoffpartikel des Kernmaterials an und polymerisieren zu einer kompakten Schale. Je nach Wandmaterial und Vernetzungsgrad können so individuelle Eigenschaften der Mikrokapseln erzielt werden.

Bei der Verkapselung unterschiedet man: Die Matrixverkapselung: Hier wird/werden der/die Wirkstoff(e) bzw. Aktivkomponente(n) mit der Hüllkomponente ("Matrix") homogen vermengt und es entsteht ein Partikel, in dem der/die Wirkstoff(e) bzw. Aktivkomponente(n) gleichmäßig verteilt ist/sind. Üblicherweise bestimmt die Freisetzung entweder die Diffusion der Aktivkomponente(n) in die Umgebung oder die Degradationsgeschwindigkeit der Matrix. Die Kern-Hülle-Verkapselung: Der/die Wirkstoff(e) bzw. Aktivkomponente(n), die den Kern bilden, werden mit einem Hüllmaterial umhüllt. Es entsteht eine echte Kapsel mit einer oder mehreren Hülle(n). Ein Hüllbruch erzeugt in der Regel eine vollständige Freisetzung des Kernmaterials in kurzer Zeit. Es ist aber auch möglich, durch die passende Wahl der Hülle eine extrem langsame Freisetzung zu erzeugen.

Aufgrund ihrer Eigenschaften werden Mikrokapseln u.a. in der Druckindustrie, der Lebensmittelindustrie (Vitamine, Aromen, Pflanzenextrakte, Enzyme, Mikroorganismen), der Agrarchemie (Düngemittel, Pflanzenschutzmittel), der Futtermittelindustrie (Mineralstoffe, Vitamine, Enzyme, Arzneimittel, Mikroorganismen), der Pharmaindustrie, der Waschmittelindustrie sowie der Kosmetikindustrie eingesetzt.

Die Verkapselung eines Wirkstoffes mit einem geeigneten Wandmaterial (Coatingmaterial) kann aus mehreren Gründen erfolgen:
- Überführung von Flüssigkeiten in eine handhabbare Pulverform (z.B. Coating von Pflanzenölen, Fetten);
- zeitlich kontrollierte Freisetzung von Substanzen (Dosierungskontrolle, Depotwirkung bei Arznei-, Pflanzenschutz- und Düngemitteln);
- Geschmacks-, Geruchs- und Farbkaschierung (z.B. bittere oder scharfe Aromastoffe);
- Schutz vor Licht, Oxidation, Hitze, Säuren oder Basen (z.B. Vitamine, Aromastoffe etc.)
- Feuchtigkeitsschutz (z.B. hygroskopische Salze oder Mineralstoffe);
- Verzögerung von Verlusten flüchtiger Komponenten (z.B. Aromastoffe);
- Verhinderung von vorzeitigen chemischen Reaktionen mit anderen Mischungskomponenten;
- besseres Handling vor oder während der Verarbeitung (Fließeigenschaften, Staubbildung);
- Schutz des Personals vor gesundheitsschädlichen oder unangenehmen Materialien (Chemikalien, Aromakonzentrate); oder
- bessere Löslichkeit/Suspendierfähigkeit durch Oberflächenmodifizierung.

Der Inhalt von Mikrokapseln kann auf verschiedene Weisen freigesetzt werden. Dabei kommen vier typische Mechanismen in Betracht:
- Die Kapseln werden mechanisch durch Zerdrücken oder Scheren zerstört. Dieser Mechanismus kommt z.B. bei Reaktionsdurchschreibepapier zur Anwendung.
- Die Kapseln werden durch Schmelzen des Wandmaterials zerstört. Nach diesem Mechanismus werden z.B. in Backmischungen Inhaltsstoffe wie Backtriebmittel oder Aromen erst während des Backvorgangs freigesetzt.
- Die Kapseln werden durch Auflösen des Wandmaterials zerstört. Dieser Mechanismus kommt z.B. bei Waschpulver zum Zuge, damit verkapselte Inhaltsstoffe wie Enzyme erst beim Waschvorgang freigesetzt werden.
- Die Kapseln bleiben erhalten, der Kapselinhalt wird durch Diffusion durch die Kapselwand nach und nach freigesetzt. Nach diesem Mechanismus kann z.B. eine langsame und gleichmäßige Freisetzung von Arzneiwirkstoffen im Körper erreicht werden.

Viele Artikel des täglichen Bedarfs wie beispielsweise Reinigungsmittel, Weichspüler, Waschpulver, Flüssigwaschmittel, Duschgele, Shampoos, Deodorants, Polylotionen etc. sind heute mit Riechstoffen bzw. Riechstoffmischungen parfümiert. Sehr häufig kommt es zu Wechselwirkungen der Riechstoffe mit anderen Bestandteilen der Formulierung oder zu einem vorzeitigen Abdampfen der leichter flüchtigen Komponenten einer Parfümierung. Dies führt in der Regel dazu, dass sich der Dufteindruck der Parfümierung im Laufe der Zeit verändert oder sogar vollständig verschwindet.

Die Mikroverkapselung solcher Riechstoffmischungen bietet die Möglichkeit, Wechselwirkungen im parfümierten Produkt oder das Abdampfen der leicht flüchtigen Duftkomponenten zu verringern bzw. vollständig zu verhindern.

Die sowohl chemisch als auch mechanisch stabilen Mikrokapseln basieren beispielsweise auf Aminoplastharzen, die als Wand- bzw. Hüllmaterial eingesetzt werden. Neben der hohen Dichtigkeit der Kapselhülle ist diese auch sehr beständig gegenüber reaktiven Chemikalien. Die Herstellung dieser Kapseln erfolgt vereinfacht dadurch, dass man zunächst unter starker Scherung und in Gegenwart von Emulgatoren eine O/W-Emulsion herstellt, welche das wasserlösliche Monomer, ein so genanntes Amin-Formaldehyd-Prekondensat, und den wasserunlöslichen, hydrophoben Wirkstoff, etwa ein Parfümöl, enthält. Die Polykondensation wird durch einen pH-Wert Wechsel initiiert, beispielsweise indem man den pH-Wert durch Säurezugabe auf etwa 3,5 einstellt. Die Polykondensate scheiden sich auf den Öltröpfchen in der Emulsion ab und hüllen sie allmählich ein. Nach Abschluss der Polykondensation ist aus der Emulsion eine Mikrokapseldispersion entstanden. Die Kapseln besitzen jedoch noch eine weiche, elastische Hülle, welche noch nicht die nötige Diffusionsstabilität und Textureigenschaften liefert. Es folgt daher ein weiterer Schritt, bei dem man die Temperatur der Mikrokapsel-Dispersion auf etwa 60 °C anhebt, was zu einer Vernetzung der Polymere in der Wandung und zur Aushärtung der Kapseln führt. Ein entsprechendes Verfahren ist beispielsweise aus der EP 2 111 214 B1 (GIVAUDAN) bekannt.

Aus der DE 23 03 866 A1 (FUJI) ist ein stabiles konzentriertes Gemisch zur Herstellung von Mikrokapseln bekannt, das (a) ein Epoxypropyltrialkylammoniumsalz und zusätzlich ein Alkylsulfosuccinat, das Alkylgruppen mit 6 bis 16 Kohlenstoffatomen besitzt, oder ein Alkylsulfosuccinamat, dessen CarbonsäureamidGruppe mit einer Alkylgruppe mit 8 bis 20 Kohlenstoffatomen substituiert ist, sowie ein mit Wasser mischbares Lösungsmittel als Komponente (b) enthält.

Gegenstand der EP 2 669 835 A1 (KOEHLER) ist ein Verfahren zur Herstellung von Mikrokapseln. Das Kennzeichen dieser Mikrokapseln ist eine spezielle Teilchengrößenverteilung, die mindestens zwei Maxima aufweist, wobei das Hauptmaximum der Teilchengröße im Bereich von 5 bis 100 µm liegt und wobei das von den Mikrokapseln, deren Teilchengröße kleiner oder gleich ein Viertel der Teilchengröße des Hauptmaximums beträgt, eingenommene Volumen größer oder gleich etwa 20 % des Gesamtvolumens der Mikrokapseln ist. Die Kapselwandung kann dabei aus einem methylierten Melamin-Formaldehydharz und/oder Harnstoff-Formaldehydharz und/oder Reaktionsprodukten von Aldehyden mit Thioharnstoff, N-Alkylharnstoff, Guanidin, Acetoguanamin, Benzoguanamin, Caprionoguanamin, Cyanamin, Dicyandiamid und/oder Alkyl-/Arylsulfonamid bestehen.

Die EP 3 238 816 A1 (SYMRISE) offenbart ein Verfahren zur Herstellung von Mikrokapseln, insbesondere Aminoplast-Mikrokapseln, bei dem die Emulgierung der wässrigen Pre-Polymer-Phase und der nicht-wässrigen Phase, die den zu verkapselnden Wirkstoff enthält, in Gegenwart mindestens eines 1,2-Diols erfolgt, was zu einer deutlichen Reduzierung der Mikrokapsel-Partikelgröße und damit zu einer Stabilisierung der Emulsion führt.

Die WO 2017/148504 (SYMRISE) betrifft ein Verfahren zur Herstellung von Riechstoffkapseln, bei dem eine Riechstoffzusammensetzung, die unmittelbar vor dem Verkapseln eine Säurezahl von maximal 5 mg KOH/g aufweist, mit natürlichen Überzugsmaterialien oder synthetischen, anionischen oder kationischen Polymeren oder Mischungen daraus verkapselt wird.

US 2017/252274 A1 offenbart ein Verfahren zur Herstellung von Mikrokapseln, welches die folgenden Schritte umfasst: Bereitstellen einer Öl Phase, umfassend ein Polyisocyanat und ein Duft Öl; Bereitstellen einer wässrigen Phase umfassend ein Tensid; Herstellung einer Öl-in-Wasser-Emulsion; Vernetzung durch Zugabe einer Mischung von Vernetzungsmitteln, ausgewählt aus einer Liste, umfassend Ethylendiamin und L-Lysin Monohydrochlorid; Härten der Mikrokapseln. In das Verfahren wird einen Katalysator verwendet. Das Tensid kann Polyvinylalkohol sein. Der Duftstoff von D1 kann eine aliphatische Carbonsäure oder ein Ester davon sein oder ein acyclisches oder cyclisches Terpenaldehyd. Die Mikrokapseln werden verwendet zur Herstellung von z.B Körperwaschmittel.

Mikrokapseln können darüber hinaus aus Acrylatmonomeren, Polyharnstoffen oder auch Biopolymeren wie Gelatine und anderen Proteinen oder anorganischen Wandmaterialien hergestellt werden. Bei den nach dem Stand der Technik hergestellten Isocyanat-basierten Mikrokapseln werden Polyisocyanate mit Guanidiniumcarbonat als Vernetzer in einem alkalischen Milieu umgesetzt.

Der Vorteil bei der Verkapselung mit Isocyanaten im Vergleich zu den Aminoplastharzen ist, dass die Mikrokapseln Formaldehyd frei sind. Nachteil der Verkapselung mit Isocyanaten und anderen Wandmaterialien ist allerdings, dass damit nur eine bedingte Anzahl an Wirkstoffen, beispielsweise Duftstoffen oder Duftölen, verkapselt werden kann und stabile Mikrokapseln liefert. Insbesondere eignet sich die Verkapselung mit Isocyanaten und anderen Wandmaterialien nicht für die Verkapselung von Duftstoffen oder Duftölen mit Aldehyd-, Carbonsäure- oder Ester-Funktionalitäten. Bei der Verkapselung bei einem alkalischen pH-Wert werden nämlich Carbonsäuren deprotoniert, oxidierte Aldehyde (Carbonsäuren) analog und Ester verseift, wodurch die erhaltene Emulsion instabil wird. Damit ist der Einsatz einer derartigen Mikroverkapselung hinsichtlich der Wirkstoffe begrenzt und somit beispielsweise nur für ein geringes Spektrum an Duftstoffen bzw. Duftölen geeignet, während eine derartige Mikroverkapselung für Duftstoffe oder Duftöle mit Aldehyd-, Carbonsäure- oder Ester-Funktionalitäten ausscheidet. Jedoch zählen gerade Riechstoffe mit Aldehyd-, Carbonsäure- oder Ester-Funktionalitäten zu den wichtigsten Vertretern bei den Duftstoffen bzw. Duftölen.

Vor diesem Hintergrund lag der vorliegenden Erfindung die komplexe Aufgabe zugrunde, ein Verfahren zur Herstellung von Mikrokapseln bereitzustellen, das einerseits eine Formaldehyd freie, für eine breite Variabilität hinsichtlich der zu verkapselnden Wirkstoffe, insbesondere von Wirkstoffen mit Aldehyd-, Carbonsäure- oder Ester-Funktionalitäten, Verkapselung ermöglicht und andererseits Mikrokapseln hervorbringt, die lagerstabil sind und ein ausgezeichnetes Freisetzungsverhalten der Wirkstoffe aufweisen.

Überraschendenderweise wurde gefunden, dass diese Aufgabe dadurch gelöst werden kann, dass die primär aus Isocyanaten gebildete Kapselhülle oder Kapselwand in einer wässrigen Emulsion in Gegenwart eines Schutzkolloids und eines Katalysators durch zwei aufeinanderfolgende Vernetzungsschritte mit jeweils einem Amin bei unterschiedlichen pH-Werten hergestellt wird. Durch eine zweistufige Vernetzung bei einem pH-Gradienten von sauer nach alkalisch ist es möglich, eine mehrschichtige (Multilayer) Kapselhülle oder Kapselwand auszubilden, wodurch selbst Wirkstoffe, wie beispielsweise Riechstoffe, mit Aldehyd-, Carbonsäure- oder Ester-Funktionalitäten verkapselt werden können.

Der verfahrenstechnische Vorteil liegt darin, dass durch die Vernetzung mit einem ersten Vernetzer bei einem sauren pH-Wert die funktionellen Gruppen der zu verkapselnden Wirkstoffe durch eine erste Polymerschicht geschützt werden, bevor der pH-Wert durch Zugabe eines weiteren, alkalischen Vernetzers angehoben wird.

### Zusammenfassung der Erfindung

Ein erster Gegenstand der Erfindung betrifft daher ein Verfahren zur Herstellung von Isocyanat-basierten oder Isothiocyanat-basierten Mikrokapseln, welches die folgenden Schritte umfasst oder aus den folgenden Schritten besteht:
(a) Bereitstellen einer internen nicht-wässrigen Phase, umfassend mindestens ein Isocyanat mit zwei oder mehreren Isocyanat-Gruppen oder mindestens ein Isothiocyanat mit zwei oder mehreren Isothiocyanat-Gruppen und einen zu verkapselnden hydrophoben Wirkstoff, der eine Aldehyd-, Carbonsäure oder Ester-Funktionalität aufweist;
(b) Bereitstellen einer externen wässrigen Phase, umfassend mindestens ein Schutzkolloid und einen Katalysator, worin das Schutzkolloid ausgewählt ist aus der Gruppe, die besteht aus Diolen, insbesondere Ethandiol, 1,2-Propandiol, 1,3-Propandiol, 1,2-Butandiol, isomeres Butandiol, 1,2-Pentandiol, 1,2-Hexandiol, 1,2-Octandiol, 1,2-Decandiol, 1,2- Dodecandiol, und Polyolen, insbesondere Triole, insbesondere Glycerin sowie dessen Ethoxylierungs- und Propoxylierungsprodukte, Trimethylolpropan sowie dessen Ethoxylierungs- und Propoxylierungsprodukte, Polyvinylalkohol (PVOH) und dessen Derivate, insbesondere Ammonium- oder Sulfonat-funktionalisierte Polyvinylalkohole, Polyphenolen, insbesondere 1,3,5-Trihydroxybenzol, Stärke oder chemisch, mechanisch und/ oder enzymatisch modifizierte Stärke, Carboxymethylcellulose und Polyvinylpyrrolidon sowie Mischungen aus den vorgenannten Verbindungen;
(c) Vermischen der internen nicht-wässrigen Phase und der externen wässrigen Phase, optional in Gegenwart eines Stabilisators und/oder Emulgators, unter Erhalt einer Öl-in-Wasser-Emulsion;
(d) erste Vernetzung durch Zugabe eines bei einem sauren pH-Wert reagierenden Amins;
(e) weitere Vernetzung durch Zugabe eines bei einem alkalischen pH-Wert reagierenden Amins unter Erhalt von Mikrokapseln;
(f) Härten der in Schritt (e) erhaltenen Mikrokapseln; und optional
(g) Abtrennen der Mikrokapseln von der Reaktionslösung und gegebenenfalls Trocknen der Mikrokapseln.

Ein weiterer Aspekt der vorliegenden Erfindung sind Mikrokapseln, die umfassen oder bestehen aus:
- einen Kern, umfassend oder bestehend aus mindestens einen hydrophoben Wirkstoff, der eine Aldehyd-, Carbonsäure- oder Ester-Funktionalität aufweist; und
- eine Kapselhülle, umfassend ein Isocyanat mit mindestens zwei oder mehreren Isocyanat-Gruppen, das in Anwesenheit eines Schutzkolloids und eines Katalysators in einem ersten Schritt durch ein bei einem sauren pH-Wert reagierendes Amin und in einem weiteren Schritt durch ein bei einem alkalischen pH-Wert reagierendes Amin vernetzt wird, wobei das Schutzkolloid ausgewählt ist aus der Gruppe, die besteht aus Diolen, insbesondere Ethandiol, 1,2-Propandiol, 1 ,3-Propandiol, 1,2-Butandiol, isomeres Butandiol, 1,2-Pentandiol, 1,2-Hexandiol, 1,2-Octandiol, 1,2-Decandiol, 1,2- Dodecandiol, und Polyolen, insbesondere Triole, insbesondere Glycerin sowie dessen Ethoxylierungs- und Propoxylierungsprodukte, Trimethylolpropan sowie dessen Ethoxylierungs- und Propoxylierungsprodukte, Polyvinylalkohol (PVOH) und dessen Derivate, insbesondere Ammonium- oder Sulfonat-funktionalisierte Polyvinylalkohole, Polyphenolen, insbesondere 1,3,5-Trihydroxybenzol, Stärke oder chemisch, mechanisch und/oder enzymatisch modifizierte Stärke, Carboxymethylcellulose und Polyvinylpyrrolidon sowie Mischungen aus den vorgenannten Verbindungen, worin die Kapselhülle umfasst oder besteht aus: eine erste Schicht aus Polyurethan und eine zweite Schicht aus Polyharnstoff, wobei sich bei der Vernetzung bei einem sauren pH-Wert eine lineare Polyharnstoff-Matrix und bei der Vernetzung bei einem alkalischen pH-Wert eine räumlich vernetzte Polyharnstoff-Matrix bildet.

Letztendlich betrifft die vorliegende Erfindung in einem weiteren Aspekt die Verwendung der erfindungsgemäßen Mikrokapseln oder von Suspensionen, die die erfindungsgemäßen Mikrokapseln umfassen, zur Herstellung von Waschmitteln, Weichspülern, Reinigungsmitteln, Scent Boostern (Duftverstärkern), Kosmetika, Körperpflegeprodukten, Agrarprodukten oder pharmazeutischen Produkten.

Diese und weitere Aspekte, Merkmale und Vorteile der vorliegenden Erfindung werden für den Fachmann aus dem Studium der folgenden detaillierten Beschreibung und der Patentansprüche ersichtlich. Dabei kann jedes Merkmal aus einem Aspekt der Erfindung in einem anderen Aspekt der Erfindung eingesetzt oder ausgetauscht werden. Ferner ist es selbstverständlich, dass die hierin enthaltenen Beispiele die Erfindung beschreiben und veranschaulichen, diese aber nicht einschränken sollen und dass insbesondere die vorliegende Erfindung nicht auf diese Beispiele beschränkt ist.

Alle Prozentangaben sind, sofern nicht anders angegeben, Gew.-%. Nummerische Beispiele, die in der Form "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

### Figuren

Figur 1 ist eine lichtmikroskopische Aufnahme der erfindungsgemäßen Mikrokapseln in 10-facher Vergrößerung. Für die lichtmikroskopische Aufnahme wurde ein Olympus BX51 verwendet.
Figur 2 ist ein Diagramm mit der Partikelgrößenverteilung (d(0,5)-Wert) von erfindungsgemäßen Mikrokapseln mit verschiedenen Parfümölen.
Figur 3 ist ein Diagramm mit der Partikelgrößenverteilung (d(0,5)-Wert) von Mikrokapseln aus dem Stand der Technik mit verschiedenen Parfümölen.
Figur 4 ist ein Diagramm mit dem Zeta-Potential von erfindungsgemäßen Mikrokapseln mit Parfümöl 4 oder 5.
Figur 5 ist ein Diagramm mit dem Zeta-Potential von Mikrokapseln aus dem Stand der Technik mit Parfümöl 1 oder 2.
Figur 6 ist ein Diagramm, das die Ergebnisse einer sensorischen Evaluierung von Mikrokapseln aus dem Stand der Technik und von erfindungsgemäßen Mikrokapseln zeigt.

### Detaillierte Beschreibung der Erfindung

In einem ersten Aspekt betrifft die vorliegende Erfindung ein Verfahren zur Herstellung von Isocyanat-basierten oder Isothiocyanat-basierten Mikrokapseln, welches die folgenden Schritte umfasst oder aus den folgenden Schritten besteht:
(a) Bereitstellen einer internen nicht-wässrigen Phase, umfassend mindestens ein Isocyanat mit zwei oder mehreren Isocyanat-Gruppen oder mindestens ein Isothiocyanat mit zwei oder mehreren Isothiocyanat-Gruppen und einen zu verkapselnden hydrophoben Wirkstoff, der eine Aldehyd-, Carbonsäure oder Ester-Funktionalität aufweist;
(b) Bereitstellen einer externen wässrigen Phase, umfassend mindestens ein Schutzkolloid und einen Katalysator, worin das Schutzkolloid ausgewählt ist aus der Gruppe, die besteht aus Diolen, insbesondere Ethandiol, 1,2-Propandiol, 1,3-Propandiol, 1,2-Butandiol, isomeres Butandiol, 1,2-Pentandiol, 1,2-Hexandiol, 1,2-Octandiol, 1,2-Decandiol, 1,2- Dodecandiol, und Polyolen, insbesondere Triole, insbesondere Glycerin sowie dessen Ethoxylierungs- und Propoxylierungsprodukte, Trimethylolpropan sowie dessen Ethoxylierungs- und Propoxylierungsprodukte, Polyvinylalkohol (PVOH) und dessen Derivate, insbesondere Ammonium- oder Sulfonat-funktionalisierte Polyvinylalkohole, Polyphenolen, insbesondere 1,3,5-Trihydroxybenzol, Stärke oder chemisch, mechanisch und/ oder enzymatisch modifizierte Stärke, Carboxymethylcellulose und Polyvinylpyrrolidon sowie Mischungen aus den vorgenannten Verbindungen;
(c) Vermischen der internen nicht-wässrigen Phase und der externen wässrigen Phase, optional in Gegenwart eines Stabilisators und/oder Emulgators, unter Erhalt einer Öl-in-Wasser-Emulsion;
(d) erste Vernetzung durch Zugabe eines bei einem sauren pH-Wert reagierenden Amins;
(e) weitere Vernetzung durch Zugabe eines bei einem alkalischen pH-Wert reagierenden Amins unter Erhalt von Mikrokapseln;
(f) Härten der in Schritt (e) erhaltenen Mikrokapseln; und
   optional
(g) Abtrennen der Mikrokapseln von der Reaktionslösung und gegebenenfalls Trocknen der Mikrokapseln.

Unter Mikrokapseln werden im Kontext der vorliegenden Erfindung Mikropartikel verstanden, die eine Kapselhülle bzw. Kapselwand und einen oder mehrere hydrophobe(n) Wirkstoff(e) als Kernmaterial im Inneren der Kapsel aufweisen.

In einem ersten Schritt des erfindungsgemäßen Verfahrens wird eine interne nicht-wässrige Phase bereitgestellt, die mindestens ein Isocyanat mit mindestens zwei oder mehreren Isocyanat-Gruppen und einen zu verkapselnden hydrophoben Wirkstoff umfasst.

Bei den erfindungsgemäß einsetzbaren polymerisationsfähigen Isocyanat-Verbindungen bzw. Isocyanat-Monomeren zur Ausbildung der Kapselhülle bzw. Kapselwand handelt es sich um solche Isocyanate oder Isothiocyanate mit mindestens zwei, drei, vier oder höher-funktionellen Isocyanat-Gruppen oder Isothiocyanat-Gruppen, insbesondere um aliphatische, cycloaliphatische, hydroaromatische, aromatische oder heterocyclische Polyisocyanate bzw. Polyisothiocyanate, deren Substitutionsprodukte sowie Mischungen aus den vorgenannten Verbindungen. Unter den Polyisocyanaten sind Diisocyanate bevorzugt. Noch mehr bevorzugt sind aromatische Diisocyanate.

Durch Verwendung von Gemischen von wenigstens zwei Isocyanat- oder Isothiocyanant-Gruppen enthaltenden verschiedenen polymerisationsfähigen Verbindungen können auch Mischpolymerisate hergestellt werden.

Beispiele für die erfindungsgemäß einsetzbaren mindestens zwei Isocyanat-Gruppen oder mindestens zwei Isothiocyanat-Gruppen enthaltende Monomere sind: Ethylendiisocyanat, Trimethylendiisocyanat, 1,4-Tetramethylendiisocyanat, 1,6-Hexamethyldiisocyanat, Ethylendiisothiocyanat, Tetramethylendiisothiocyanat, Hexamethylendiisothiocyanat, Cyclobutan-1,3-diisocyanat, Cyclohexan-1,3-diisocyanat, Cyclohexan-1,4-diisocyanat, 1,3-Phenylendiisocyanat, 1,4-Phenylendiisocyanat, Gemische von 1,3-Phenylendiisocyanat und 1,4-Phenylendiisocyanat, p-Phenylendiisothiocyanat, Xylylen-1,4-diisothiocyanat, 2,4-Toluylendiisocyanat, 2,6-Toluylendiisocyanat, Gemische von 2,4-Toluylendiisocyanat und 2,6-Toluylendiisocyanat, Xylylen-1,4-diisocyanat, Xylylen-1,3-diisocyanat sowie Gemische von Xylylen-1,4-diisocyanat und Xylylen-1,3-diisocyanat, 2,4-Hexahydrotoluylendiisocyanat, 2,6-Hexahydrotoluylendiisocyanat, Gemische von 2,4-Hexahydrotoluylendiisocyanat und 2,6-Hexahydrotoluylendiisocyanat, Hexahydro-1,3-phenylendiisocyanat, Hexahydro-1,4-phenylendiisocyanat, Gemische von Hexahydro-1,4-phenylendiisocyanat und Hexahydro-1,4-phenylendiisocyanat, 1,3-Diisocyanatobenzol, 1,3,5-Trimethylbenzol-2,4-diisocyanat, 1,3,5-Triisopropylbenzol-2,4-diisocyanat, Diphenylmethan-4,4'-diisocyanat, 3,3'-Dimethyldiphenylmethan-4,4'-diisocyanat, 4,4'-Diphenylpropandiisocyanat, Naphthylen-1,4-diisocyanat, Naphthylen-1,5-diisocyanat, Triphenylmethan-4,4',4"-triisocyanat, Toluylen-2,4,6-triisocyanat, Dimethyldiphenylmethan-2,2',5,5'-tetraisocyanat oder Mischungen der vorgenannten Verbindungen.

Als wenigstens zwei Isocyanat- bzw. Isothiocyanat-Gruppen aufweisende polymerisationsfähige Verbindungen werden vorzugsweise großtechnisch hergestellte Di- und Polyisocyanate bevorzugt, beispielsweise TDI: Toluylendiisocyanat (Isomerengemisch von 2,4- und 2,6-Toluylendiisocyanat im Verhältnis von 80:20), HDI: Hexamethylendiisocyanat-(1,6), IPDI: Isophorondiisocyanat oder DMDI: Diphenylmethan-4,4'-diisocyanat.

Weitere Ausgangsisocyanate, die in dem erfindungsgemäßen Verfahren zum Einsatz kommen, sind: Diisocyanate wie 1,4-Diisocyanatobutan, 1,6-Diisocyanatohexan, 1,5-Diisocyanato-2,2-dimethylpentan, 2,2,4- und 2,4,4-Trimethyl-1,6-diisocyanatohexan, 1,10-Diisocyanatodecan, 1,3- und 1,4-Diisocyanatocyclohexan, 1-Isocyanato-3,3,5-trimethyl-5-isocyanatomethylcyclohexan (Isophorondiisocyanat), 4,4'-Diisocyanatodicyclohexylmethan, 2,4- und 2,6-Diisocyanato-methylcyclohexan und deren Gemische. Prinzipiell können auch aromatische Isocyanate, z.B. Toluylendiisocyanate oder 4,4'-Diisocyanatodiphenylmethan eingesetzt werden.

Anteilig können auch Polyisocyanate, die durch Modifizierung der oben genannten Diisocyanate oder deren Mischungen nach bekannten Verfahren darstellbar sind und z.B. Uretdion-, Urethan-, Isocyanurat-, Biuret- und/oder Allophanat-Gruppen enthalten, mit eingesetzt werden.

Der Anteil der Isocyanat-Komponente in der internen nicht-wässrigen Phase liegt in einem Bereich von 1 bis 8 Gew.-%, vorzugsweise in einem Bereich von 3 bis 7 Gew.-%, bezogen auf das Gesamtgewicht der nicht-wässrigen Phase. Am meisten bevorzugt wird die Isocyanat-Komponente in der internen nicht-wässrigen Phase in einem Bereich von 5 bis 6 Gew.-%, bezogen auf das Gesamtgewicht der nicht-wässrigen Phase, verwendet.

Die erfindungsgemäßen Mikrokapseln sind so ausgestaltet, dass sie ein Kernmaterial aus mindestens einem hydrophoben Wirkstoff, insbesondere einem hydrophoben Duftstoff bzw. einem hydrophoben Duftöl, einem Pestizid, einem Biozid, einem Insektizid, einer Substanz aus der Gruppe der Repellentien, Lebensmittel-Additiven, kosmetischen Wirkstoffen, pharmazeutischen Wirkstoffen, Farbstoffen, Agrochemikalien, Farbstoffen, Leuchtfarben, optischen Aufhellern, Lösungsmitteln, Wachsen, Silikonölen, Schmierstoffen, sowie Mischungen aus den vorgenannten Wirkstoffen aufweisen, d.h. solange dieser hinreichend wasserunlöslich oder sich nicht mit der Wasserphase vermischt, da sich andernfalls keine Emulsion bilden und keine Abscheidung des Polymers auf der Tröpfchenoberfläche erfolgen kann.

In einer bevorzugten Variante des ersten Aspekts der vorliegenden Erfindung kommen als Wirkstoffe insbesondere hydrophobe Duftstoffe bzw. Duftöle, Aromen oder auch biogene Prinzipien in Betracht.

In einer bevorzugten Ausführungsform weisen die erfindungsgemäßen Mikrokapseln ein Kernmaterial in Form eines hydrophoben Einzel-Duftstoffes bzw. Einzel-Riechstoffes auf, wobei das Kernmaterial mindestens einen Einzel-Duftstoff bzw. Einzel-Riechstoff oder Mischungen daraus umfasst, ausgewählt aus einer oder mehreren der folgenden Gruppen:
- Kohlenwasserstoffe, wie z. B. 3-Caren; a-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
- Aliphatische Alkohole, wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3,4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
- Aliphatische Aldehyde und deren Acetale, wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
- Aliphatische Ketone und deren Oxime, wie z. B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on;
- Aliphatische schwefelhaltige Verbindungen, wie z. B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
- Aliphatische Nitrile, wie z. B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
- Aliphatische Carbonsäuren und deren Ester, wie z. B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
- Acyclische Terpenalkohole, wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-I-ol; sowie deren Formiate, Acetate, Propionate, Isobutyrate, Butyrate, Isovalerianate, Pentanoate, Hexanoate, Crotonate, Tiglinate, 3-Methyl-2-butenoate;
- Acyclische Terpenaldehyde und -ketone, wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
- Cyclische Terpenalkohole, wie z. B. Menthol; Isopulegol; a-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
- Cyclische Terpenaldehyde und -ketone, wie z. B. Menthon; Isomenthon; 8-Mercaptomenthan-3-on; Carvon; Campher; Fenchon; α-Iοnοn; beta-Ionon; α-n-Methylionon; beta-n-Methylionon; α-Isomethylionon; beta-Isomethylionon; α-Iron; ß-Iron; α-Damascenon; beta-Damascenon; gamma-Damascenon; d-Damascenon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; α-Sinensal; beta-Sinensal; acetyliertes Cedernholzöl (Methylcedrylketon);
- Cyclische Alkohole, wie z. B. 4-tert-Butylcyclohexanol; 3,3,5-Trimethylcyclohexanol; 3-Isocamphylcyclohexanol; 2,6,9-Trimethyl-(Z2,Z5,E9)-cyclododecatrien-1-ol; 2-Isobutyl-4-methyltetrahydro-2H-pyran-4-ol; aus der Gruppe der cycloaliphatischen Alkohole wie z. B.3,3,3-Trimethyl-cyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
- Cyclische und cycloaliphatische Ether, wie z. B. Cineol; Cedrylmethylether; Cyclododecylmethylether; (Ethoxymethoxy)cyclododecan; α-Cedrenepoxid; 3a,6,6,9a-Tetramethyldodecahydronaphtho[2,1-b]furan; 3a-Ethyl-6,6,9a-trimethyl-dodecahydronaph-tho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
- Cyclische Ketone, wie z. B. 4-tert-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert-Pentylcyclohexanon; 5-Cyclohexadecen-1-on; 6,7-Dihydro-1,1,2,3,3-pentamethyl-4(5H)-indanon; 9-Cycloheptadecen-1-on; Cyclopentadecanon; Cyclohexadecanon;
- Cycloaliphatische Aldehyde, wie z. B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
- Cycloaliphatische Ketone, wie z. B. 1-(3,3-Dimethylcyclohexyl)-4-penten-1-on; 1-(5,5-Dimethyl-2-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert-Butyl-(2,4-dimethyl-3-cyclohexen-1-yl)keton;
- Ester cyclischer Alkohole, wie z. B. 2-tert-Butylcyclohexylacetat; 4-tert-Butylcyclo-hexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5- bzw. -6-indenylacetat; 4,7-Methano-3a,4,5,6,7,7a-hexahydro-5- bzw. -6-indenylpropionat; 4,7-Methano-3a,4,5,6,7,7a-hexahydro-5- bzw. -6-indenylisobutyrat; 4,7-Methanooctahydro-5-bzw. -6-indenylacetat;
- Ester cycloaliphatischer Carbonsäuren, wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
- Aromatische Kohlenwasserstoffe, wie z. B. Styrol und Diphenylmethan;
- Araliphatische Alkohole, wie z. B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenyl-ethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
- Ester von araliphatischen Alkoholen und aliphatischen Carbonsäuren, wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; α-Trichlormethylbenzylacetat; a,a-Dimethylphenylethylacetat; a,a-Dimethylphenylethylbutyrat; Cinnamylacetat; 2-Phenoxyethylisobutyrat; 4-Methoxybenzylacetat;
- Araliphatischen Ether, wie z. B. 2-Phenylethylmethylether; 2-Phenylethylisoamylether; 2-Phenylethyl-1-ethoxyethylether; Phenylacetaldehyddimethylacetal; Phenylacetaldehyd-diethylacetal; Hydratropaaldehyd-dimethylacetal; Phenylacetaldehyd-glycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
- Aromatische und araliphatische Aldehyde, wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)-propanal; 2-Methyl-3-(4-tert-butylphenyl)propanal; 3-(4-tert-Butylphenyl)propanal; Zimtaldehyd; a-Butylzimtaldehyd; a-Amylzimtaldehyd; a-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
- Aromatische und araliphatische Ketone, wie z. B. Acetophenon; 4-Methylacetophenon; 4-Methoxyacetophenon; 4-tert-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert-Butyl-1,1-dimethyl-4-indanylmethyl-keton; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
- Aromatische und araliphatische Carbonsäuren und deren Ester, wie z. B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethyl-phenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
- Stickstoffhaltige aromatische Verbindungen, wie z. B. 2,4,6-Trinitro-1,3-dimethyl-5-tert-butylbenzol; 3,5-Dinitro-2,6-dimethyl-4-tert-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiffsche Basen von Methylanthranilat mit 7-Hydroxy-3,7-dimethyloctanal, 2-Methyl-3-(4-tert-butylphenyl)propanal oder 2,4-Dimethyl-3-cyclohexencarbaldehyd; 6-Isopropylchinolin; 6-Isobutylchinolin; 6-sec-Butylchinolin; Indol; Skatol; 2-Methoxy-3-isopropylpyrazin; 2-Isobutyl-3-methoxypyrazin; 4-(4,8-Dimethyl-3,7-nonadienyl)-pyridin;
- Phenole, Phenylether und Phenylester, wie z. B. Estragol; Anethol; Eugenol; Eugenylmethylether; Isöugenol; Isöugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat; aus der Gruppe der heterocyclischen Verbindungen wie z. B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
- Lactone, wie z. B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid, 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin;
sowie die Stereoisomere, Enantiomere, Stellungsisomere, Diastereomere, cis/trans-Isomere bzw. Epimere der zuvor genannten Substanzen.

In einer alternativen Ausführungsform kommen als Kernmaterial Duftöle bzw. Parfümöle zum Einsatz. Dabei handelt es sich um Zusammensetzungen, die mindestens einen Riechstoff enthalten. Solche Kompositionen, insbesondere Duftöle bzw. Parfümöle, umfassen vorzugsweise zwei, drei, vier, fünf, sechs, sieben, acht, neun, zehn oder mehr Riechstoffe. Die Duftöle bzw. Parfümöle sind vorzugsweise ausgewählt aus der Gruppe der Extrakte aus natürlichen Rohstoffen, wie Etherische Öle, Concretes, Absolüs, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos-Absolü; Bayöl; Beifussöl; Benzöresin; Bergamotteöl; Bienenwachs-Absolü; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolü; Castoreum-Absolü; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolü; Eichenmoos-Absolü; Elemiöl; Estragonöl; Eukalyptus-Citriodoraöl; Eukalyptusöl; Fenchelöl; Fichtennadelöl; Galbanumöl; Galbanumresin; Geraniumöl; Grapefruitöl; Guajakholzöl; Gurjunbalsam; Gurjunbalsamöl, Helichrysum-Absolü; Helichrysumöl; Ingweröl; Iriswurzel-Absolü; Iriswurzelöl; Jasmin-Absolü; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolü; Labdanumresin; Lavandin-Absolü; Lavandinöl; Lavendel-Absolü; Lavendelöl; Lemongrasöl; Liebstocköl; Limetteöl destilliert; Limetteöl gepresst; Linalööl; Litsea-Cubeba-öl; Lorbeerblätteröl; Macisöl; Majoranöl; Mandarinenöl; Massoirindenöl; Mimosa-Absolü; Moschuskörneröl; Moschustinktur; Muskateller-Salbei-öl; Muskatnussöl; Myrrhen-Absolü; Myrrhenöl; Myrtenöl; Nelkenblätteröl; Nelkenblütenöl; Neroliöl; Olibanum-Absolü; Olibanumöl; Opopanaxöl; Orangenblüten-Absolü; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolü; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-Tree-öl; Terpentinöl; Thymianöl; Tolubalsam; Tonka-Absolü; Tuberosen-Absolü; Vanilleextrakt; Veilchenblätter-Absolü; Verbenaöl; Vetiveröl; Wacholderbeeröl; Weinhefenöl; Wermutöl; Wintergrünöl; Ylangöl; Ysopöl; Zibet-Absolü; Zimtblätteröl; Zimtrindenöl; sowie Fraktionen davon bzw. daraus isolierte Inhaltsstoffe.

Von den zuvor genannten Einzel-Duftstoffen bzw. Einzel-Riechstoffe, die im Sinne der vorliegenden Erfindung verkapselt werden können, kommen zum Einsatz Duftstoffe bzw. Riechstoffe, die eine Aldehyd-, Carbonsäure- oder Ester-Funktionalität aufweisen.

Aldehydische Duftstoffe bzw. Riechstoffe, welche auch die entsprechenden Acetale sowie Ester und Lactone umfassen, lassen sich in die folgenden Gruppen unterteilen, nämlich
(i) aliphatische Aldehyde und deren Acetale;
(ii) cycloaliphatische Aldehyde;
(iii) aromatische oder araliphatische Aldehyde;
(iv) aliphatische, aromatische oder araliphatische Ester; und
(v) Lactone;
sowie jeweils deren Gemische.

Die zuvor genannten Duftstoffe bzw. Riechstoffe mit Aldehyd-, Carbonsäure- oder Ester-Funktionalität sowie Mischungen daraus sind ausgewählt aus einer oder mehreren der folgenden Gruppen:
- Aliphatische Aldehyde und deren Acetale, wie z. B. Hexanal; Heptanal; Octanal; Nonanal; Decanal; Undecanal; Dodecanal; Tridecanal; 2-Methyloctanal; 2-Methylnonanal; (f)-2-Hexenal; (Z)-4-Heptenal; 2,6-Dimethyl-5-heptenal; 10-Undecenal; (f)-4-Decenal; 2- Dodecenal; 2,6,10-Trimethyl-5,9-undecadienal; Heptanal-diethylacetal; 1,1-Dimethoxy-2,2,5-trimethyl-4-hexen; Citronellyloxyacetaldehyd;
- Cycloaliphatische Aldehyde, wie z. B. 2,4-Dimethyl-3-cyclohexencarbaldehyd; 2-Methyl-4-(2,2,6-trimethyl-cyclohexen-I-yl)-2-butenal; 4-(4-Hydroxy-4-methylpentyl)-3-cyclohexencarbaldehyd; 4-(4-Methyl-3-penten-1-yl)-3-cyclohexencarbaldehyd;
- Aromatische und araliphatische Aldehyde, wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)- propanal; 2-Methyl-3-(4-te/t-butylphenyl)propanal; 3-(4-te/t-Butylphenyl)propanal; Zimtaldehyd; α-Butylzimtaldehyd; α-Amylzimtaldehyd; α-Hexylzimtaldehyd; 3-Methyl-5-phenylpentanal; 4-Methoxybenzaldehyd; 4-Hydroxy-3-methoxybenzaldehyd; 4-Hydroxy-3ethoxybenzaldehyd; 3,4-Methylendioxybenzaldehyd; 3,4-Dimethoxybenzaldehyd; 2-Methyl- 3-(4-methoxyphenyl)-propanal; 2-Methyl-3-(4-methylendioxyphenyl)-propanal;
- Aliphatische Carbonsäureester, wie z. B. (E)- und (Z)-3-Hexenylformiat; Ethylacetoacetat; Isoamylacetat; Hexylacetat; 3,5,5-Trimethylhexylacetat; 3-Methyl-2-butenylacetat; (f)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3- Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
- Ester cyclischer Alkohole, wie z. B. 2-te/t-Butylcyclohexylacetat; 4-te/t-Butylcyclohexylacetat; 2-ieri-Pentylcyclohexylacetat; 4-te/t-Pentylcyclohexylacetat; Decahydro-2-naphthylacetat; 3-Pentyltetrahydro-2/-/- pyran-4-ylacetat; Decahydro-2,5,5,8a-tetramethyl-2-naphthylacetat; 4,7-Methano-3a,4,5,6,7,7a-hexahydro-5- bzw. -6-indenylacetat; 4,7-Methano-3a,4,5,6,7,7a-hexahydro-5- bzw. -6-indenylpropionat; 4,7-Methano-3a,4,5, 6,7,7a-hexahydro-5- bzw. -6-indenylisobutyrat; 4,7-Methanooctahydro-5- bzw. - 6-indenylacetat;
- Ester von araliphatischen Alkoholen und aliphatischen Carbonsäuren, wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; a-Trichlormethylbenzylacetat; α,α-Dimethylphenylethylacetat;α,α-Dimethylphenylethylbutyrat; Cinnamylacetat; 2-Phenoxyethylisobutyrat; 4-Methoxybenzylacetat;
- Ester cycloaliphatischer Carbonsäuren, wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6- tetramethyl-2-cyclohexencarboxylat; Ethyl-2-methyl-I,3-dioxolan-2-acetat;
- Aromatische und araliphatische Carbonsäureester, wie z. B. Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat.

Nachfolgend sind Aldehyde, Acetale, Ester und Lactone mit ihren kommerziellen Bezeichnungen aufgelistet, die im Sinne des erfindungsgemäßen Verfahrens als Vertreter der Gruppen (i) bis (v) besonders bevorzugt sind:
Aldehyde: 2-Methylpentanal; Aldehyd C12 MNA HM; Aldehyde C 4; Aldehyde C 5; Aldehyde C 6; Aldehyde C 7; Aldehyde C 8; Aldehyde C 9; Aldehyde C 10; Aldehyde C 11 ISO; Aldehyde C 11 MOA PURE; Aldehyde C 11 UNDECANAL; Aldehyde C 11 UNDEYLENIC; Aldehyde C 12; ; Aldehyde C 12 MNA; Aldehyde C 13; ALDEHYDE MADARINE; AMYL CINNAMIC ALDEHYDE ALPHA; ANISALALDEHYDE-O; ANISYL ALDEHYDE; BENZALDEHYDE NAT.; BERGAMAL; BORONAL; BOURGENOAL; CAMPHONELIC ALDEHYDE; CITRAL; CITRONELLAL HM; CITRONELLYL OXYACET ALDEHYDE; CITRYLAL; CITROYLAL E HM; CORTEX ALDEHYDE; CORTEX ALDEHYDE 50 PCT PEMOSA; CROTONIC ALDEHYDE; CUMINAL ALDEHYDE; CYCLAMEN ALDEHYDE; DECADIENAL TRANS, TRANS-2,4, DECANAL CIS-4; DECANAL TRANS-2; DECANAL TRANS-2 NAT; DECANAL TRANS-4; DECANAL-9,1; DODECANIENAL 2,6; DODECANAL TRANS-2; DUPICAL; EPOXYDECENAL-4,5-2 10% TRI; ETHYL HEXANAL; FARENAL^{®}; FLORHYDRAL; GERALDEHYDE; HELIONAL; HELIOPAN; HELIOTROPIN; HEPTADIENAL TRANS, TRANS,2-4; HEPTENAL CIS-4; HEPTENAL TRANS-2; HEXENAL TRANS-2; HEXYL CINNAMIC ALDEHYDE ALPHA; HYDRATROPIC ALDEHYDE; HYDROXY CITRONELLAL; INTRELEVEN ALDEHYDE SPEC.; ISONONYL ALDEHYDE; ISOVALERIC ALDEHYDE; LEMON ALDEHYDE H&R JS I; LILIAL; LINOLAL; LYRAL; MAJANTAL; MANDRINAL; MANDRAINE ALDEHYDE 10% IN TEC BHT; MEFRANAL; MELONAL^{®}; METHODY CITRONELLAL; METHYL BUTYRALDEHYDE; METHYL CINNAMIC ALDEHYDE ALPHA; METHYL PHENYLPENTENAL-4,2,2; METHYL THIO PROPANAL-3; METHYL TRIDECANAL-12 10% VT; METHYL-3-BUTEN-2-AL; METHYL-5-PHENYL-2-HEXEN-2-AL; MUGENAL 50 DPG; NEOCYCLO CITRAL; NONADIENAL; TRANS, CIS-2,6; NONENAL CIS-6; NONENAL TRANS-2; ONCIDAL^{®} 3/060251; PENTENAL TRANS-2; PERILLA ALDEHYDE; PHENYLACET ALDEHYDE; PHENYLBUTENAL TRANS-2,2; PHENYLPROPYL ALDEHYDE; PINOACET ALDEHYDE; PROFRANESAL; PROPIONALALDEHYD 2-(P-TOLYL); PROPIONIC ALDEHYDE; PS-IRALDEIN X NEU; SAFRANAL; SALICYLIC ALDEHYDE FG; SILVIAL; TETRAHYDRO CITRAL; TIGLIC ALDEHYDE-2,2; TOLYL ALDEHYDE PARA FG; TRIDECENAL TRANS-2; TRIFERNAL; UNDECADIENAL-2,4; UNDECENAL TRANS-2; VERNALALDEHYDE; VERTOCITRAL; VERTOMUGAL; VERTIPRENAL; VETRAL ROH; ZIMTALDEHYD NAT. HM; Acetale: FLOROPAL; HEPTANAL DIETHYL ACETAL; NONANDIENAL DIETHYL ACETAL; OKOUMAL; PHENYLACET ALD. GLYCERIN ACETAL; PHENYLACETALDEYHDEDIMETHYLACETAL; Ester: JASMAL; JESSEMAL; KHARISMAL; TIRAMISONE^{®}.

In einer weiteren Variante des erfindungsgemäßen Verfahrens lassen sich auch Aromastoffe als Kernmaterial in Form eines Einzelaromas verkapseln, wobei das Kernmaterial mindestens einen Einzelaromastoff oder Mischungen daraus umfasst.

Typische Beispiele für Aromen, die im Sinne der Erfindung verkapselt werden können, sind ausgewählt aus der Gruppe, die besteht aus: Acetophenon; Allylcapronat; alpha-Ionon; beta-Ionon; Anisaldehyd; Anisylacetat; Anisylformiat; Benzaldehyd; Benzothiazol; Benzylacetat; Benzylalkohol; Benzylbenzoat; beta-Ionon; Butylbutyrat; Butylcapronat; Butylidenphthalid; Carvon; Camphen; Caryophyllen; Cineol; Cinnamylacetat; Citral; Citronellol; Citronellal; Citronellylacetat; Cyclohexylacetat; Cymol; Damascon; Decalacton; Dihydrocumarin; Dimethylanthranilat; Dimethylanthranilat; Dodecalacton; Ethoxyethylacetat; Ethylbuttersäure; Ethylbutyrat; Ethylcaprinat; Ethylcapronat; Ethylcrotonat; Ethylfuraneol; Ethylguajakol; Ethylisobutyrat; Ethylisovalerianat; Ethyllactat; Ethylmethylbutyrat; Ethylpropionat; Eucalyptol; Eugenol; Ethylheptylat; 4-(p-Hydroxyphenyl)-2-butanon; gamma-Decalacton; Geraniol; Geranylacetat; Geranylacetat; Grapefruitaldehyd; Methyldihydrojasmonat (z.B. Hedion^{®}); Heliotropin; 2-Heptanon; 3-Heptanon; 4-Heptanon; trans-2-Heptenal; cis-4-Heptenal; trans-2-Hexenal; cis-3-Hexenol; trans-2-Hexensäure; trans-3-Hexensäure; cis-2-Hexenylacetat; cis-3-Hexenylacetat; cis-3-Hexenylcapronat; trans-2-Hexenylcapronat; cis-3-Hexenylformiat; cis-2-Hexylacetat; cis-3-Hexylacetat; trans-2-Hexylacetat; cis-3-Hexylformiat; para-Hydroxybenzylaceton; Isoamylalkohol; Isoamylisovalerianat; Isobutylbutyrat; Isobutyraldehyd; Isoeugenolmethylether; Isopropylmethylthiazol; Laurinsäure; Leavulinsäure; Linalool; Linalooloxid; Linalylacetat; Menthol; Menthofuran; Methylanthranilat; Methylbutanol; Methylbuttersäure; 2-Methylbutylacetat; Methylcapronat; Methylcinnamat; 5-Methylfurfural; 3,2,2-Methylcyclopentenolon; 6,5,2-Methylheptenon; Methyldihydrojasmonat; Methyljasmonat; 2-Methylmethylbutyrat; 2-Methyl-2-Pentenolsäure; Methylthiobutyrat; 3,1-Methylthiohexanol; 3-Methylthiohexylacetat; Nerol; Nerylacetat; trans,trans-2,4-Nonadienal; 2,4-Nonadienol; 2,6-Nonadienol; 2,4-Nonadienol; Nootkaton; delta-Octalacton; gamma-Octalacton; 2-Octanol; 3-Octanol; 1,3-Octenol; 1-Octylacetat; 3-Octylacetat; Palmitinsäure; Paraldehyd; Phellandren; Pentandion; Phenylethylacetat; Phenylethylalkohol; Phenylethylalkohol; Phenylethylisovalerianat; Piperonal; Propionaldehyd; Propylbutyrat; Pulegon; Pulegol; Sinensal; Sulfurol; Terpinen; Terpineol; Terpinolen; 8,3-Thiomenthanon; 4,4,2-Thiomethylpentanon; Thymol; delta-Undecalacton; gamma-Undecalacton; Valencen; Valeriansäure; Vanillin; Acetoin; Ethylvanillin; Ethylvanillinisobutyrat (3-Ethoxy-4-isobutyryloxybenzaldehyd); 2,5-Dimethyl-4-hydroxy-3(2H)-furanon und dessen Abkömmlinge (vorzugsweise Homofuraneol (2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon); Maltol und Maltol-Abkömmlinge (vorzugsweise Ethylmaltol); Cumarin und Cumarin-Abkömmlinge; gamma-Lactone (vorzugsweise gamma-Undecalacton, gamma-Nonalacton, gamma-Decalacton); delta-Lactone (vorzugsweise 4-Methyldeltadecalacton, Massoilacton, Deltadecalacton, Tuberolacton); Methylsorbat; Divanillin; 4-Hydroxy-2(oder 5)-ethyl-5(oder 2)-methyl-3(2H)furanon; 2-Hydroxy-3-methyl-2-cyclopentenon; 3-Hydroxy-4,5-dimethyl-2(5H)-furanon; Essigsäureisoamylester; Buttersäureethylester; Buttersäure-n-butylester; Buttersäureisoamylester; 3-Methyl-buttersäureethylester; n-Hexansäureethylester; n-Hexansäureallylester; n-Hexansäure-n-butylester; n-Octansäureethylester; Ethyl-3-methyl-3-phenylglycidat; Ethyl-2-trans-4-cis-decadienoat; 4-(p-Hydroxyphenyl)-2-butanon; 1,1-Dimethoxy-2,2,5-trimethyl-4-hexan; 2,6-Dimethyl-5-hepten-1-al; Phenylacetaldehyd; 2-Methyl-3-(methylthio)furan; 2-Methyl-3-furanthiol; bis(2-Methyl-3-furyl)disulfid; Furfurylmercaptan; Methional; 2-Acetyl-2-thiazolin; 3-Mercapto-2-pentanon; 2,5-Dimethyl-3-furanthiol; 2,4,5-Trimethylthiazol; 2-Acetylthiazol; 2,4-Dimethyl-5-ethylthiazol; 2-Acetyl-1-pyrrolin; 2-Methyl-3-ethylpyrazin; 2-Ethyl-3,5-dimethylpyrazin; 2-Ethyl-3,6-dimethylpyrazin; 2,3-Diethyl-5-methylpyrazin; 3-Isopropyl-2-methoxypyrazin; 3-Isobutyl-2-methoxypyrazin; 2-Acetylpyrazin; 2-Pentylpyridin; (E,E)-2,4-Decadienal; (E,E)-2,4-Nonadienal; (E)-2-Octenal; (E)-2-Nonenal; 2-Undecenal; 12-Methyltridecanal; 1-Penten-3-on; 4-Hydroxy-2,5-dimethyl-3(2H)-furanon; Guajakol; 3-Hydroxy-4,5-dimethyl-2(5H)-furanon; 3-Hydroxy-4-methyl-5-ethyl-2(5H)-furanon; Zimtaldehyd; Zimtalkohol; Methylsalicylat; Isopulegol sowie die vorliegend nicht explizit genannten Stereoisomere, Enantiomere, Stellungsisomere, Diastereomere, cis/trans-Isomere bzw. Epimere dieser Substanzen.

Am meisten bevorzugt kommen Duftstoffe bzw. Riechstoffe oder Aromastoffe bei der Herstellung der Isocyanat-basierten Mikrokapseln zur Anwendung, die ausgewählt sind aus der Gruppe, die besteht aus: AGRUMEX LC; AGRUNITRIL; ALDEHYD C11 UNDECYLENIC; ALDEHYD C12 LAURIN; ALDEHYD C12 MNA; ALDEHYD C14 SOG; ALDEHYD C16 SOG.; ALLYLAMYLGLYCOLAT; ALLYLCAPRONAT; ALLYLCYCLOHEXYLPROPIONAT; ALLYLHEPTYLAT; AMBROCENIDE^{®} 10 TEC; AMBROCENIDE^{®} Krist. 10% IPM; AMBROXIDE; ANETHOL NAT. EX STERNANIS; ANISALDEHYD REIN; APRIFLOREN^{®}; BENZYLACETON; BENZYLSALICYLAT; BORNEOL L/ISOBORNEOL 65/35; BUCCOBLAETTEROEL; CITRONELLOL 950; CLONAL; CYCLOHEXYLSALICYLAT; CYMOL PARA SUPRA; DAMASCONE DELTA; DIHYDROMYRCENOL; DIMETHYLBENZYLCARBINYLBUTYRAT; DYNASCONE; ETHYLENBRASSYLAT; ETHYLMETHYLBUTYRAT-2; ETHYLSAFRANAT; EUCALYPTOL NAT.; EUKALYPTUSOEL GLOBULUS 80/85%; EUGENOL NAT.; FARENAL^{®}; FENCHELOEL AROMA TYP SUESS NAT.; FILBERTONE 10% IPM; FILBERTONE; FLOROPAL; GALBASCONE; GERANIOL 60; GLOBANONE^{®}; HEDION; HERBAFLORAT; HERBANATE; HERBYLPROPIONAT; HEXENYLACETAT CIS-3; HEXENYLSALICYLAT CIS-3; HEXYLACETAT; HEXYLACETAT S; HEXYLISOBUTYRAT; HEXYLSALICYLAT; ISOAMYLBUTYRAT; ISOBORNYLACETAT; ISOPROPYLMETHYLBUTYRAT-2; ISORALDEIN 70; JAVANOL; KAMPFER DL; KRESOLMETHYLETHER P(CR< 10 PPM); LEMONILE; LIGUSTRAL; LILIAL; LINALOOL; MANZANATE; MELONAL; METHYLHEPTINCARBONAT; METHYLOCTINCARBONAT; MUSCENONE; NEOCYCLOCITRAL; NEROLIN BROMELIA; NEROLIN YARA YARA KRIST.; NEROLIONE; NORLIMBANOL; ORANGENOEL; ORIVONE; OZONIL; PATCHOULIOEL ENTF.; PFLANZENOEL TRIGLYCERID; PHELLANDREN FRAKTION EX EUKALYPTUSOEL; PHENIRAT^{®}; PHENYLETHYLACETAT; ROSENOXID HIGH CIS; SANDRANOL^{®}; STYROLYLACETAT; SULTANENE^{®}; TERPINEN GAMMA; TETRAHYDROLINALOOL; TIMBERSILK; TRIETHYLCITRAT; UNDECAVERTOL; VERTOCITRAL; VERTOFIX; YSAMBER^{®} K und Mischungen aus den vorgenannten Wirkstoffen.

In einer weiteren Variante des erfindungsgemäßen Verfahrens lassen sich auch biogene Prinzipien als Kernmaterial verkapseln, wobei das Kernmaterial mindestens ein biogenes Prinzip oder Mischungen daraus umfasst.

Unter biogenen Prinzipien sind Wirkstoffe mit biologischer Aktivität zu verstehen, beispielsweise Tocopherol, Tocopherolacetat, Tocopherolpalmitat, Ascorbinsäure, Carnotin, Carnosin, Koffein, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, sowie Vitaminkomplexe.

Bei dem erfindungsgemäßen Verfahren zur Herstellung der Isocyanat-basierten Mikrokapseln wird als erstes das oben beschriebene mindestens zwei Isocyanat- oder Isothiocyanat-Gruppen enthaltende Monomer in einem inerten Lösungsmittel oder Lösungsmittelgemisch zusammen mit dem zu verkapselnden hydrophoben Wirkstoff gelöst. Die so gebildete organische hydrophobe, ölige Phase bildet die interne nicht-wässrige Phase.

Als inerte Lösungsmittel für die interne nicht-wässrige Phase seien genannt: chloriertes Diphenyl, chloriertes Paraffin, Pflanzenöle wie beispielsweise Baumwollsamenöl, Erdnussöl, Palmöl, Trikresylphosphat, Silikonöl, Dialkylphthalate, Dialkyladipate, teilhydrierte Terphenyl, alkyliertes Biphenyl, alkyliertes Naphthalin, Diarylether, Arylalkylether und höher alkyliertes Benzol, Benzylbenzoat, Isopropylmyristat sowie beliebige Mischungen dieser hydrophoben Lösungsmittel und Mischungen einzelner oder mehrerer dieser hydrophoben Lösungsmittel mit Kerosin, Paraffinen und/oder Isoparaffinen. Bevorzugt werden Pflanzenöl-Triglyceride, Benzylbenzoat oder Isopropylmyristat als Lösungsmittel für die Bereitstellung der internen nicht-wässrigen Phase verwendet.

Die interne nicht-wässrige Phase kann beispielsweise 20 bis 50 Gew.-%, vorzugsweise 25 bis 45 Gew.-% und noch mehr bevorzugt 33 bis 40 Gew.-% einzukapselnden hydrophoben Wirkstoff, 1 bis 8 Gew.-%, vorzugsweise 3 bis 7 Gew.-% und noch mehr bevorzugt 5 bis 6 Gew.-% Isocyanat und ergänzend zu 100 Gew.% hydrophobe Lösungsmittel enthalten, bezogen auf das Gesamtgewicht der internen nicht-wässrigen Phase.

Die interne nicht-wässrige Phase, welche mindestens ein Isocyanat mit zwei oder mehreren Isocyanat-Gruppen und mindestens einen hydrophoben Wirkstoff umfasst, wird in einer externen wässrigen oder hydrophilen Phase unter Bildung einer Öl-in-Wasser-Emulsion emulgiert.

Die externe wässrige Phase umfasst mindestens ein Schutzkolloid und mindestens einen Katalysator.

Bei einem Schutzkolloid handelt es sich um eine Verbindung, die bei Fällungsreaktionen, d. h. bei Reaktionen, in denen aus einer homogenen flüssigen Phase eine feste Phase abgeschieden wird, ein Zusammenklumpen (Agglomerieren, Aggregation, Ausflocken, Koagulieren) der Primärpartikel verhindert. Das Schutzkolloid lagert sich mit seinem hydrophoben Teil an die Primärpartikel an und wendet seinen polaren, d. h. hydrophilen Molekülteil, der wässrigen Phase zu. Durch diese Anlagerung an der Grenzfläche erniedrigt es die Grenzflächenspannung und verhindert die Agglomeration der Primärteilchen. Zudem stabilisiert es die Emulsion und begünstigt dabei die Bildung vergleichsweise kleinerer Tröpfchen und damit auch entsprechender Mikrokapseln.

Das in dem erfindungsgemäßen Verfahren gemäß dem ersten Aspekt der vorliegenden Erfindung verwendete Schutzkolloid ist ausgewählt aus der Gruppe, die besteht aus Diolen, insbesondere Ethandiol, 1,2-Propandiol, 1,3-Propandiol, 1,2-Butandiol, isomeres Butandiol, 1,2-Pentandiol, 1,2-Hexandiol, 1,2-Octandiol, 1,2-Decandiol, 1,2-Dodecandiol, und Polyolen, insbesondere Triole, wie beispielsweise Glycerin sowie dessen Ethoxylierungs- und Propoxylierungsprodukte, Trimethylolpropan sowie dessen Ethoxylierungs- und Propoxylierungsprodukte, Polyvinylalkohol und dessen Derivate, wie beispielsweise Ammonium- oder Sulfonat-funktionalisierte Polyvinylalkohole, Polyphenolen, vorzugsweise 1,3,5-Trihydroxybenzol, Stärke, insbesondere Stärke aus Weizen, Kartoffeln, Mais, Reis, Tapioka oder Hafer, oder chemisch, mechanisch und/oder enzymatisch modifizierte Stärke, sowie Mischungen aus den vorgenannten Verbindungen.

Weitere Schutzkolloide, die der externen wässrigen Phase zur Stabilisierung der Emulsion zugesetzt werden können, sind vorzugsweise Carboxymethylcellulose oder Polyvinylpyrrolidon, das beispielsweise unter der Bezeichnung Luviskol^{®} im Handel ist, sowie Mischungen aus den vorgenannten Verbindungen.

Am meisten bevorzugt kommen als Schutzkolloid Polyole, Polyphenole oder Stärke, insbesondere modifizierte Stärke, zum Einsatz. Besonders bevorzugt wird zur Herstellung der erfindungsgemäßen Mikrokapseln Polyvinylalkohol oder seine Ammonium-Derivate, 1,3,5-Trihydroxybenzol oder modifizierte Stärke als Schutzkolloid verwendet.

Die zuvor genannten Verbindungen haben eine Doppelfunktion: zum einen reagieren sie mit dem/den Isocyanat(en), wodurch sich stabile Mikrokapseln ausbilden und zum anderen wirken sie als Schutzkolloid, indem sie die Agglomeration der festen Partikel verhindern, die Emulsion stabilisieren und somit die Bildung von kleinen Tröpfchen begünstigen.

Überraschend und als besonders vorteilhaft hat es sich erwiesen, wenn in der externen wässrigen Phase eines der o.g. Schutzkolloide in Kombination mit Stärke oder modifizierter Stärke als weiterem Schutzkolloid verwendet wird. Durch eine derartige Kombination wird eine Reaktion zwischen dem Schutzkolloid und dem/den Isocyanat(en) begünstigt.

Am meisten bevorzugt ist die kombinierte Verwendung eines Schutzkolloids mit modifizierter Stärke. Die Verwendung einer der zuvor genannten Kombinationen hat zum einen den Vorteil, dass die feste Phase in der Formulierung nicht ausflocken kann und die Emulsion stabilisiert wird und zum anderen führt die gemeinsame Verwendung zu besonders stabilen Mikrokapseln.

Als besonders vorteilhafte Kombination hat sich ein Polyphenol, vorzugsweise 1,3,5-Trihydroxybenzol, mit Stärke oder modifizierter Stärke erwiesen. Am allermeisten bevorzugt ist eine Kombination aus 1,3,5-Trihydroxybenzol mit modifizierter Stärke.

Das 1,3,5-Trihydroxybenzol wird als Hydroxylgruppendonor eingesetzt; um bei Schutzkolloiden ohne Hydroxylgruppen entsprechende Gruppen zu liefern und somit einen Gewinn an Stabilität zu gewährleisten.

Die verwendete Menge des Schutzkolloids oder die verwendete Menge einer Kombination von Schutzkolloiden liegt in einem Bereich von 1 bis 8 Gew.-%, vorzugsweise in einem Bereich von 2 bis 4 Gew.-%, noch mehr bevorzugt in einem Bereich von 3 bis 4 Gew.-%, bezogen auf das Gesamtgewicht der externen wässrigen Phase.

Das Verhältnis von Schutzkolloid in der externen wässrigen Phase zu Isocyanat bzw. Isothiocyanat in der internen nicht-wässrigen Phase liegt in einem Bereich von 1 : 5 bis 1 : 100, vorzugsweise in einem Bereich von 1 : 10 bis 1 : 75, noch mehr bevorzugt in einem Bereich von 1 : 25 bis 1 : 50.

Bei dem Katalysator, der in dem erfindungsgemäßen Verfahren in der externen wässrigen Phase verwendet wird, handelt es sich vorzugsweise um Diazobicyclo[2.2.2]octan (DABCO). DABCO, auch Triethylendiamin (TEDA) genannt, ist ein bicyclisches, tertiäres Amin. DABCO wird als Katalysator zur Herstellung von Polyurethan-Kunststoffen verwendet. Das tertiäre Amin mit freien Elektronenpaaren begünstigt die Reaktion zwischen dem Isocyanat in der internen nicht-wässrigen Phase und dem Schutzkolloid in der externen wässrigen Phase.

Die Menge, in der der Katalysator der externen wässrigen Phase zugesetzt wird, liegt in einem Bereich von 0,001 bis 1 Gew.-%, vorzugsweise in einem Bereich von 0,02 bis 0,75 Gew.-% und besonders bevorzugt in einem Bereich von 0,05 bis 0,5 Gew.-%, bezogen auf das Gesamtgewicht der externen wässrigen Phase. Bei träg ablaufender Polymerisation kann der Katalysator jedoch auch erhöht werden.

Die Emulsionsbildung (bei flüssigen Wirkstoffen) bzw. Suspensionsbildung (bei festen Wirkstoffen), d.h. das Emulgieren bzw. Suspendieren der internen nicht-wässrigen bzw. öligen Phase mit der externen wässrigen bzw. hydrophilen Phase erfolgt unter hoher Turbulenz bzw. starker Scherung, wobei die Stärke der Turbulenz bzw. der Scherung den Durchmesser der erhaltenen Mikrokapseln bestimmt. Die Herstellung der Mikrokapseln kann dabei kontinuierlich oder diskontinuierlich erfolgen. Mit steigender Viskosität der wässrigen Phase oder mit fallender Viskosität der öligen Phase nimmt in der Regel die Größe der Kapseln ab.

Überraschend wurde festgestellt, dass sich beim Emulgieren bzw. Suspendieren der internen nicht-wässrigen Phase in der externen wässrigen Phase in Anwesenheit des Schutzkolloids, vorzugsweise eines Polyols, an den Grenzflächen der emulgierten bzw. suspendierten zu verkapselnden hydrophoben Partikel, die den Kern der erfindungsgemäßen Mikrokapsel bilden, durch Grenzflächenpolymerisation eine Kapselhülle bzw. Kapselwand in Kernnähe ausbildet. Die Ausbildung beruht auf der Polyadditionsreaktion des Polyisocyanats mit dem Schutzkolloid, vorzugsweise eines Polyols, unter Bildung einer Kapselhülle bzw. Kapselwand aus Polyurethan gemäß der nachfolgenden Formel:

O=C=N-R-N=C=O + -(CH₂-CH(OH)-)ₙ- →-(R-O-CO-NH-R-NH-CO-O-)ₙ

Dies zeigt sich durch Gasentwicklung und Freisetzung von Kohlendioxid.
Es kann vorteilhaft sein, wenn zur Herstellung der Isocyanat-basierten Mikrokapseln gemäß der vorliegenden die externe wässrige Phase gegebenenfalls weitere Schutzkolloide und/oder Stabilisatoren und/oder Emulgierhilfen entweder gelöst oder dispergiert enthält. Solche Mittel können, bezogen auf das Gesamtgewicht der wässrigen Phase, beispielweise in Mengen von 0,05 bis 5 Gew.-%, vorzugsweise von 0,1 bis 4 Gew.-% zugesetzt werden.

Vorzugsweise enthält die externe wässrige Phase zudem Stabilisatoren, die die Emulsion/Slurry stabilisieren, um eine Entmischung der internen nicht-wässrigen (öligen) Phase und der externen wässrigen Phase zu verhindern. Die bevorzugten Stabilisatoren zur Herstellung der Isocyanat-basierten Mikrokapseln gemäß der vorliegenden Erfindung sind vor allem Acrylcopolymere, die über Sulfonatgruppen verfügen. Ebenfalls geeignet sind Copolymere von Acrylamiden und Acrylsäure, Copolymere von Alkylacrylaten und N-Vinylpyrrolidon wie beispielsweise LUVISKOL^{®} K15, K30 oder K90 (BASF); Natriumpolycarboxylate, Natriumpolystyrolsulfonate, Vinyl- und Methylvinylether-Maleinsäureanhydrid-Copolymere sowie Ethylen-, Isobutylen- oder Styrol-Maleinsäureanhydrid-Copolymere, mikrokristalline Cellulose, die beispielweise unter der Bezeichnung VIVAPUR^{®} im Handel ist, Diutan Gum, Xanthan Gum oder Carboxymethylcellulosen.

Die Einsatzmenge der Stabilisatoren kann im Bereich von 0,01 bis 10 Gew.-% und insbesondere im Bereich von 0,1 bis 3 Gew.-%, jeweils bezogen auf die externe wässrige Phase, liegen.

Optional werden in dem erfindungsgemäßen Verfahren Emulgatoren, vorzugsweise O/W-Emulgatoren, eingesetzt, die eine homogene Verteilung der Öltröpfchen der internen nicht-wässrigen Phase in der externen wässrigen Phase ermöglichen und die Emulsion stabilisieren. Ähnliches gilt für die Aufmischung von festen, nicht löslichen Wirkstoffen in der externen wässrigen Phase, um die so erhaltene Suspension zu stabilisieren.

Als Emulgatoren kommen beispielweise nichtionogene Tenside aus mindestens einer der folgenden Gruppen in Betracht:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Rizinusöl und/oder gehärtetes Rizinusöl;
- Anlagerungsprodukte von 15 bis 60 Mol Ethylenoxid an Rizinusöl und/oder gehärtetes Rizinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid, vorzugsweise Cremophor^{®};
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin;
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate; Block-Copolymere z.B. Polyethylenglycol-30 Dipolyhydroxystearate; Polymeremulgatoren, z.B. Pemulen-Typen (TR-1,TR-2) von Goodrich oder Cosmedia^{®} SP von Cognis;
- Polyalkylenglycole sowie Glycerincarbonat.

Typische anionische Emulgatoren, die in dem erfindungsgemäßen Verfahren zur Herstellung der Isocyanat-basierten Mikrokapseln verwendet werden können, sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

Weiterhin können in dem erfindungsgemäßen Verfahren zur Herstellung der Isocyanat-basierten Mikrokapseln als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C8/18-Alkyl- oder Acylgruppe im Molekül mindestens eine freie Aminogruppe und mindestens eine -COOH- oder -SO3H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12/18-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

Die Emulgatoren können der externen wässrigen Phase in einer Menge von etwa 0,5 bis etwa 10 Gew.-% und vorzugsweise etwa 1 bis etwa 5 Gew.-%, jeweils bezogen auf das Gesamtgewicht der externen wässrigen Phase, zugesetzt werden.

Das erfindungsgemäße Verfahren zur Herstellung der Mikrokapsel-Dispersion kann beispielsweise nach der "Inline"-Technik erfolgen. Dabei werden mittels einer Zwangsdosierpumpe die interne nicht-wässrige Phase und die externe wässrige Phase zunächst getrennt einer Emulgierturbine zugeführt, kurz vor dem Einlauf in die Emulgierturbine vereinigt oder in der Emulgierturbine vereinigt, bei einem Durchlaufvolumen von 1200 bis 1500 1/h.

Das Gewichtsverhältnis von internen nicht-wässriger Phase zu externer wässriger Phase liegt vorzugsweise in einem Bereich von 10 : 90 bis 60 : 40, vorzugsweise in einem Bereich von 30 : 70 bis 50 : 50.

Darüber hinaus kann das erfindungsgemäße Verfahren zur Herstellung der Isocyanat-basierten Mikrokapseln auch in üblichen Dispersionsapparaturen bzw. Emulgiervorrichtungen erfolgen.

Der Prozess des Emulgierens bzw. Suspendierens in dem erfindungsgemäßen Verfahren wird vorteilhafter Weise für eine Zeit von 0,5 bis 10 Minuten, vorzugsweise von 0,75 bis 4 Minuten und ganz besonders bevorzugt von 1 bis 2,5 Minuten durchgeführt, bis sich eine Kapselgröße von 10 bis 50 µm ± 5 µm eingestellt hat.

In einem darauffolgenden Schritt des erfindungsgemäßen Verfahrens wird ebenfalls unter Rühren bei einer Rührgeschwindigkeit von 1000 bis 1500 Upm, vorzugsweise bei einer Rührgeschwindigkeit von 1200 Upm, eine erste Vernetzung des Materials der Kapselhülle bzw. Kapselwand durchgeführt. Die erste Vernetzung erfolgt durch Zugabe eines bei einem sauren pH-Wert reagierenden Amins, um die das Kernmaterial einschließende Kapselhülle zu vernetzen und zu stabilisieren.

Besonders vorteilhafte Ergebnisse zur Kapselhüllen-Bildung werden erzielt, wenn die erste Vernetzung mit dem Amin bei einem sauren pH-Wert von 2 bis 7 erfolgt, vorzugsweise bei einem pH-Wert von 2 bis 6 erfolgt und am meisten bevorzugt bei einem leicht sauren pH-Wert von 3 bis 5. Hierzu wird der externen wässrigen Phase eine Säure, beispielsweise Ameisensäure oder Essigsäure, zugesetzt und ein pH-Wert in den o.g. Bereichen eingestellt.

Die Durchführung der ersten Vernetzung in einem sauren pH-Wert-Bereich hat gegenüber der Vernetzung in einem basischen Milieu den Vorteil, dass die hydrophoben Wirkstoffe mit Aldehyd-, Carbonsäure- oder Ester-Funktionalitäten bei den ersten Reaktionsschritten der Vernetzung des Isocyanats bzw. Isothiocyanats nicht verseifen.

Damit wird verhindert, dass der zu verkapselnde Wirkstoff oder die zu verkapselnden Wirkstoffe, beispielsweise ein Parfümöl, chemisch verändert wird/werden und damit einhergehend einerseits ein Verlust der Wirkstoffe auftritt und andererseits die Emulsion instabil wird.

Noch mehr bevorzugt wird der Schritt der ersten Vernetzung bei einer Temperatur von 20 °C bis 30 °C durchgeführt.

Das bei einem sauren pH-Wert reagierende Amin ist ausgewählt aus der Gruppe, die besteht aus basischen Aminosäuren und deren Hydrochloriden, insbesondere Lysin-Hydrochlorid oder Ornithin-Hydrochlorid. Am meisten bevorzugt ist als Vernetzungsmittel Lysin-Hydrochlorid.

Das erste Vernetzungsmittel wird der Emulsion entweder als solches, beispielsweise als Feststoff, oder in Form einer wässrigen Lösung zugesetzt.

Das erste Vernetzungsmittel ist in der wässrigen Lösung in einer Konzentration von 0,5 bis 2 Mol/l, vorzugsweise von 1 Mol/l, enthalten. Die Lösung weist einen pH-Wert von 4 bis 7 auf.

Die erste Vernetzung in dem erfindungsgemäßen Verfahren wird für eine Zeitdauer von etwa 10 Minuten bis 20 Minuten durchgeführt, vorzugsweise für eine Zeitdauer von 12 bis 18 Minuten und am meisten bevorzugt für eine Zeitdauer von etwa 15 Minuten.

Dem ersten Vernetzungsschritt folgt ein weiterer Vernetzungsschritt des Materials der Kapselhülle bzw. Kapselwand durch Zugabe eines bei einem alkalischen pH-Wert reagierenden Amins unter Bildung einer Polyharnstoff-Matrix.

Besonders vorteilhafte Ergebnisse zur Kapselbildung wurden erzielt, wenn die weitere Vernetzung mit dem bei einem alkalischen pH-Wert reagierenden Amin bei einem alkalischen pH-Wert von 7 bis 14 erfolgt, vorzugsweise bei einem alkalischen pH-Wert von 7 bis 10 und am meisten bevorzugt bei einem alkalischen pH-Wert von 7 bis 8.

Noch mehr bevorzugt wird der Schritt der weiteren Vernetzung bei einer Temperatur von 35 °C bis 50 °C durchgeführt.

Das bei einem alkalischen pH-Wert reagierende Amin ist ausgewählt aus der Gruppe, die besteht aus der Di-, Tri- und Polyamine und Guanidiniumcarbonat. Am meisten bevorzugt ist als weiteres Vernetzungsmittel Guanidiniumcarbonat.

Das weitere Vernetzungsmittel wird der Emulsion entweder als solches, beispielsweise als Feststoff, oder in Form einer wässrigen Lösung zugesetzt.

Das weitere Vernetzungsmittel ist in der wässrigen Lösung in einer Konzentration von 0,5 bis 2 Mol/l, vorzugsweise von 1 Mol/l, enthalten. Die Lösung weist einen pH-Wert von 7 bis 14, vorzugsweise einen pH-Wert von 12 auf.

Durch Zugabe des bei einem alkalischen pH-Wert reagierenden Amins wird der pH-Wert der Emulsion zu höheren pH-Werten verschoben. Der pH-Wert des Endproduktes, d.h. der finalen Kapselslurry, liegt bei 5 bis 8, vorzugsweise bei einem pH-Wert von 6 bis 7.

Wesentliches Merkmal der Herstellung von Isocyanat-basierten Mikrokapseln gemäß der vorliegenden Erfindung ist somit, dass die erste Vernetzung bei einem sauren pH-Wert durchgeführt wird und die daran anschließende weitere Vernetzung bei einem alkalischen pH-Wert durchgeführt wird.

Durch den pH-Wert-Gradienten bildet sich bei der ersten Vernetzung, vorzugsweise bei einem sauren pH-Wert, zunächst eine Kapselhülle mit einer linearen Polyharnstoff-Matrix. Bei der anschließenden weiteren Vernetzung, vorzugsweise bei einem basischen pH-Wert, wird die Kapselhülle weiter vernetzt und es bildet sich eine räumlich vernetzte Polyharnstoff-Matrix.

Während der beiden Vernetzungsschritte wird die Rührleistung zurückgenommen, beispielsweise auf eine Rührgeschwindigkeit von etwa 800 bis 1200 Upm, um die sich bildenden Mikrokapseln nicht gleich wieder zu zerschlagen.

Nach der vollständigen Vernetzung und Ausbildung der Kapselhülle bzw. Kapselwand liegen die nach dem erfindungsgemäßen Verfahren hergestellten Kapseln als rohe Mikrokapseln in Form einer wässrigen Dispersion bzw. einer Slurry vor.

Nach der Vernetzung weisen die Mikrokapseln in der Slurry noch eine flexible Hülle auf, die keine sonderliche Stabilität besitzt und daher leicht aufbricht. Zu diesem Zweck wird eine Härtung der Hülle durchgeführt. Die Härtung wird durch eine Temperaturänderung ausgelöst, indem man die Slurry schrittweise bis auf eine Temperatur von 70 °C, vorzugsweise auf eine Temperatur im Bereich von 60 bis 65 °C, anhebt. Die Härtung wird üblicherweise über eine Zeitdauer von 2 bis 4 Stunden durchgeführt.

Zusätzlich vorteilhaft ist es, der externen wässrigen Phase Substanzen zur Härtung zuzusetzen. Für diesen Zweck werden natürliche pflanzliche Gerbstoffe vom Typ der Tannine eingesetzt, bei denen es sich chemisch betrachtet um Proanthocyanidine handelt, wie sie in dikotylen Stauden, Sträuchern und Blättern besonders in den Tropen und Subtropen zu finden sind. Die Terpene weisen in der Regel molekulare Gewichte im Bereich von 500 bis 3.000 KDa auf. Ein bevorzugtes Beispiel für ein geeignetes Tannin ist das Corigallin. Zur Härtung wird eine wässerige Zubereitung der Tannine der die rohen Mikrokapseln enthaltenden wässrigen Dispersion zugegeben. Üblicherweise setzt man die Tannine in Mengen von etwa 0,1 bis etwa 2 Gew.-% und vorzugsweise von etwa 0,5 bis etwa 1,5 Gew.-%, bezogen auf die Mikrokapseln, zu.

Ein für die Einsetzbarkeit der Mikrokapseln wichtiges Kriterium ist das Gewichtsverhältnis von Kernmaterial zu Kapselwandmaterial. Während einerseits ein möglichst hoher Anteil an Kernmaterial angestrebt wird, um einen möglichst hohen Nutzwert der Kapseln zu ermöglichen, ist es auf der anderen Seite notwendig, dass die Kapsel einen noch hinreichenden Anteil an Kapselwandmaterial aufweisen, damit die Stabilität der Kapseln gewährleistet ist.

Erfindungsgemäß hat es ich als besonders vorteilhaft erwiesen, dass die Mikrokapseln so ausgestaltet sind, dass die Mikrokapseln ein Gewichtsverhältnis von Kernmaterial zu Kapselwandmaterial aufweisen, das bei 50 : 50 bis 90 : 10, vorzugsweise bei 70 : 30 bis 80 : 20 liegt.

Nach der Härtung liegen die nach dem erfindungsgemäßen Verfahren hergestellten Mikrokapseln als Dispersion in Wasser vor, die auch als Mikrokapsel-Dispersion bezeichnet wird. In dieser Form sind die Mikrokapseln grundsätzlich bereits verkaufsfähig; zu Konservierungszwecken empfiehlt es sich jedoch, sie zu trocknen.

Grundsätzlich kommen dafür Verfahren wie die Lyophilisierung in Frage, bevorzugt ist jedoch eine Sprühtrocknung beispielsweise in der Wirbelschicht. Dabei hat es sich als vorteilhaft erwiesen, der Dispersion bei Temperaturen von etwa 20 bis etwa 50 °C und vorzugsweise etwa 40 °C weitere Polysaccharide, vorzugsweise Dextrine und insbesondere Maltodextrine zuzugeben, die den Trockenprozess unterstützen und die Kapseln während dieses Vorgangs schützen. Dabei kann die Einsatzmenge der Polysaccharide etwa 50 bis etwa 150 Gew.-% und vorzugsweise etwa 80 bis etwa 120 Gew.-% bezogen auf die Kapselmasse in der Dispersion betragen.

Die Sprühtrocknung selbst kann in konventionellen Sprühanlagen kontinuierlich oder batchweise durchgeführt werden, wobei die Einlasstemperatur bei etwa 170 bis etwa 200 °C und vorzugsweise etwa 180 bis 185 °C liegt und die Austrittstemperatur etwa 70 bis etwa 80 °C und vorzugsweise etwa 72 bis 78 °C beträgt.

Es hat sich gezeigt, dass mit dem erfindungsgemäßen Verfahren die Verkapselung von hydrophoben Wirkstoffen, insbesondere von hydrophoben Wirkstoffen, die eine Aldehyd-, Carbonsäure- oder Ester-Funktionalität aufweisen, möglich ist. Die Mikrokapseln zeichnen sich durch eine hervorragende Stabilität und ein hervorragendes Freisetzungsvermögen aus.

Überraschenderweise hat sich herausgestellt, dass sich mit dem erfindungsgemäßen Verfahren darüber hinaus Mikrokapseln mit einer verringerten Menge an Material der Kapselhülle bzw. Kapselwand um bis zu 50 % herstellen lassen, ohne dass dadurch eine Einbuße oder ein Verlust der Stabilität der erhaltenen Mikrokapseln auftritt, wie dies in den nachfolgenden Ausführungsbeispielen gezeigt wird. Dadurch können Mikrokapseln mit einer verringerten Menge an Ausgangssubstanz Isocyanat bei gleichbleibender Menge an zu verkapselndem Wirkstoff hergestellt werden.

Außerdem sind die mit dem erfindungsgemäßen Verfahren hergestellten Mikrokapseln Formaldehyd frei.

In einem weiteren Aspekt betrifft die vorliegenden Erfindung Mikrokapseln, die nach dem erfindungsgemäßen Verfahren herstellt werden.

Wie aus den nachfolgenden Daten ersichtlich ist, entstehen bei der Emulgierung in Gegenwart eines Schutzkolloids und eines Katalysators und einer zweistufigen Vernetzung des Isocyanat-basierten Hüllmaterials mit einem bei einem sauren pH-Wert reagierenden Amin und mit einem bei einem alkalischen pH-Wert reagierenden Amin stabile Mikrokapseln. Insbesondere entstehen stabile Mikrokapseln mit einem Kernmaterial, das einen hohen Anteil an Wirkstoffen mit Aldehyd-, Carbonsäure- oder Ester-Funktionalität aufweist.

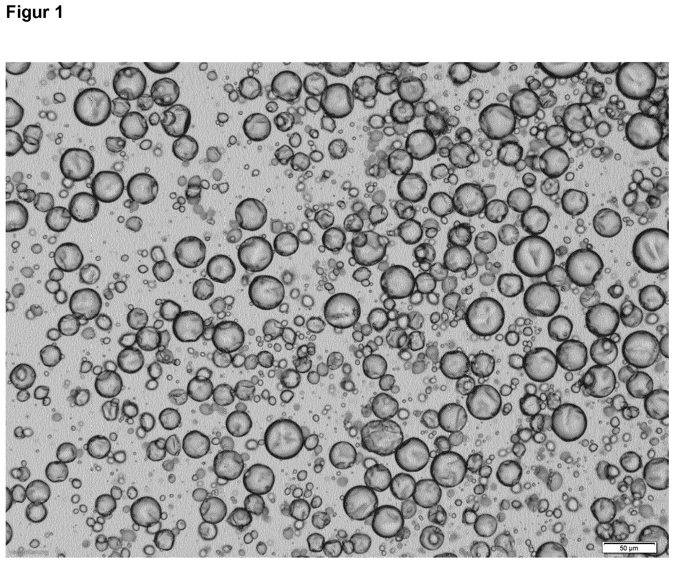

Die erfindungsgemäßen Mikrokapseln sind in Figur 1 in 10-facher Vergrößerung veranschaulicht. Der Aufbau der Kapselhülle kann durch Lichtmikroskopie beobachtet werden. Während zu Beginn des Herstellungsverfahrens Öltröpfchen erkennbar sind, sind nach dem Vernetzen, Aufheizen und Abkühlen eingedellte Mikrokapseln zu erkennen.

Die nach dem erfindungsgemäßen Verfahren hergestellten Mikrokapseln lassen sich über die folgenden Parameter ihrer Größenverteilung charakterisieren:
- d(0,1)-Wert: 10 % der Kapseln sind kleiner als dieser Wert;
- d(0,5)-Wert: 50 % der Kapseln sind größer, 50 % der Kapseln sind kleiner als dieser Wert; und
- d(0,9)-Wert: 10 % der Kapseln sind größer als dieser Wert.

Zur Bestimmung der Partikelgröße werden im Rahmen eines dynamischen Prozesses die erfindungsgemäßen Mikrokapseln in Wasser dispergiert und dann die Partikelgröße mittels Laserbeugung bestimmt. Abhängig von der Größe der Kapsel wird der Laserstrahl unterschiedlich gebrochen und kann so zu einer Größe umgerechnet werden. Dafür wurde die Mie-Theorie verwendet. Für die Partikelmessung wurde ein MALVERN Mastersizer 3000 verwendet.

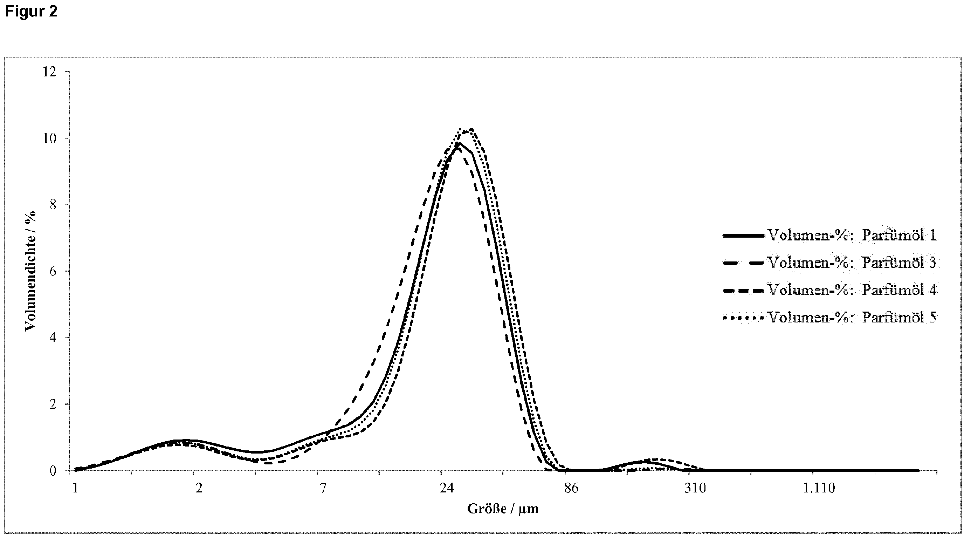

Die erfindungsgemäßen Mikrokapseln sind dadurch gekennzeichnet, dass sie eine Partikelgrößenverteilung bei einem d(0,5)-Wert von 10 - 50 µm, vorzugsweise einen d(0,5)-Wert von 20 bis 30 µm aufweisen, wie dies in Figur 2 veranschaulicht ist, und eine Partikelgrößenverteilung bei einem d(0,9)-Wert von 10 bis 120 µm, vorzugsweise einen d(0,9)-Wert von 60 bis 90 µm aufweisen.

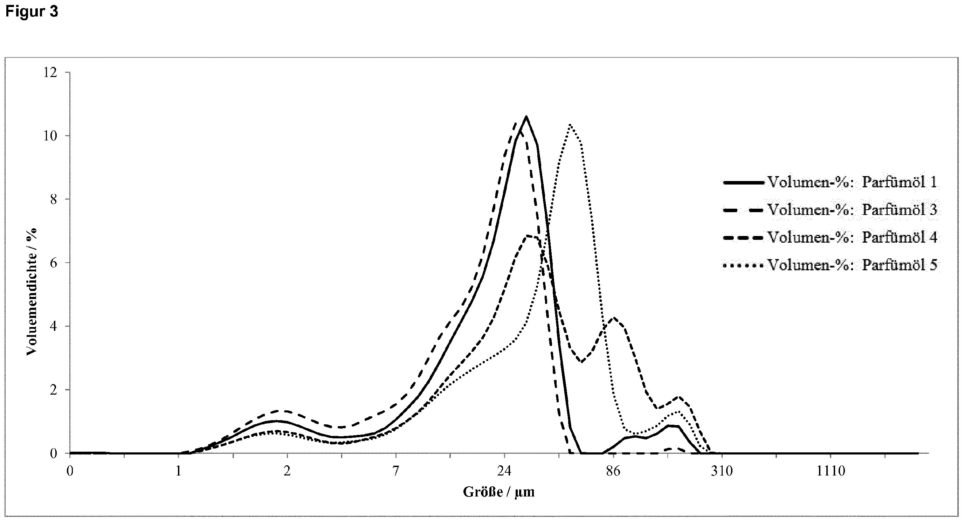

Figur 3 zeigt die Partikelgrößenverteilung (d(0,5)-Wert) von Mikrokapseln, die nach dem Stand der Technik hergestellt wurden. Für die Parfümöle 4 und 5 fällt auf, dass eine Größeneinstellung nicht möglich war.

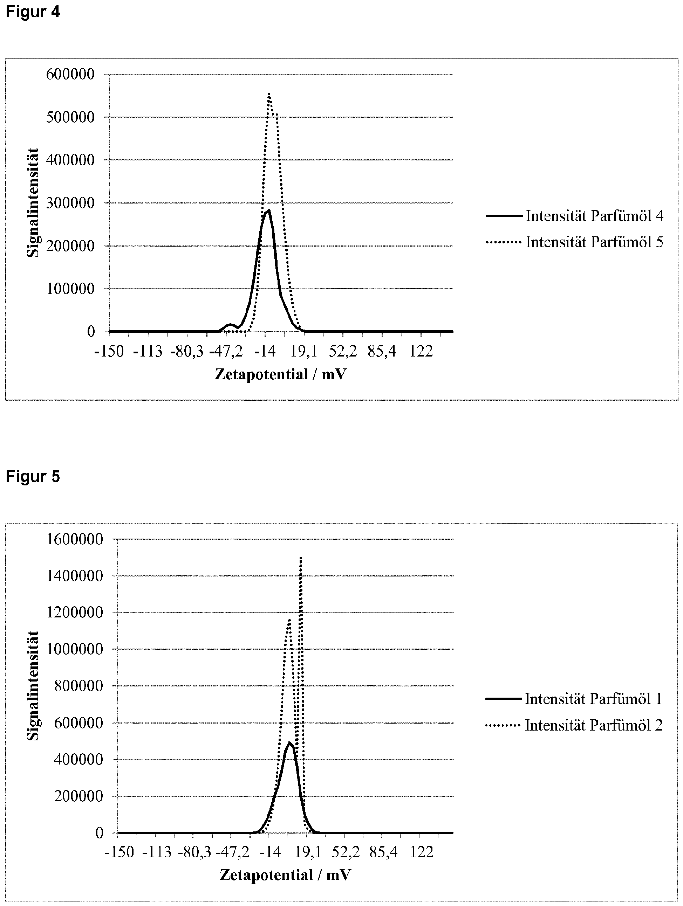

Die nach dem erfindungsgemäßen Verfahren herstellten Mikrokapseln zeichnen sich weiter dadurch aus, dass sie ein Zetapotential aufweisen, das für die erfindungsgemäßen Mikrokapseln charakteristisch bei - 5 bis - 15 mV liegt, wie dies in den Figuren 4 und 5 veranschaulicht ist. Vorzugsweise liegt das Zetapotential bei - 10 mV. Jede Mikrokapsel hat ein Zetapotential. Das Zetapotential ist unabhängig von dem zu verkapselnden Wirkstoff, insbesondere vom Parfümöl, hängt aber gleichwohl vom Polymer der Kapselhülle ab. Aufgrund der Änderung des Zetapotentials bei der Herstellung der erfindungsgemäßen Mikrokapseln ist es möglich nachzuweisen, dass sich ein neues Polymer bzw. eine Hülle gebildet hat, da sich die Oberfläche und somit auch die Oberflächenladung (Zetapotential) der Partikel in der Emulsion ändert. Die erfindungsgemäßen Mikrokapseln, die ohne Katalysator hergestellt werden, weisen ein Zetapotential von - 27 mV auf. Mikrokapseln aus dem Stand der Technik haben ein Zetapotential von ± 0 mV. Im Rahmen der Messungen wurde die Smoluchowski-Theorie angewendet. Für die Messung des Zetapotentials wurde ein MALVERN Zetasizer nanoZS verwendet.

Unabhängig von der Signalintensität, welche durch die Dosierung der Mikrokapseln im Dispergiermedium kommt, ist aus den Figuren 4 und 5 klar erkennbar, dass das Zetapotential für die erfindungsgemäßen Mikrokapseln bei - 5 bis 0 mV liegt, da der Einsatz des Katalysators und die damit einhergehende Beschleunigung der Polyurethan-Bildungsreaktion kennzeichnend für das abweichende Zetapotential von - 27 mV ist.

Ein weiterer Aspekt der vorliegenden Erfindung sind daher Mikrokapseln, die umfassen oder bestehen aus:
- einen Kern, umfassend oder bestehend aus mindestens einen hydrophoben Wirkstoff, der eine Aldehyd-, Carbonsäure- oder Ester-Funktionalität aufweist; und
- eine Kapselhülle, umfassend ein Isocyanat mit zwei oder mehreren Isocyanat-Gruppen, das in Anwesenheit eines Schutzkolloids und eines Katalysators in einem ersten Schritt durch ein bei einem sauren pH-Wert reagierendes Amin und in einem weiteren Schritt durch ein bei einem alkalischen pH-Wert reagierendes Amin vernetzt wird, wobei das Schutzkolloid ausgewählt ist aus der Gruppe, die besteht aus Diolen, insbesondere Ethandiol, 1,2-Propandiol, 1,3-Propandiol, 1,2-Butandiol, isomeres Butandiol, 1,2-Pentandiol, 1,2-Hexandiol, 1,2-Octandiol, 1,2-Decandiol, 1,2- Dodecandiol, und Polyolen, insbesondere Triole, insbesondere Glycerin sowie dessen Ethoxylierungs- und Propoxylierungsprodukte, Trimethylolpropan sowie dessen Ethoxylierungs- und Propoxylierungsprodukte, Polyvinylalkohol(PVOH) und dessen Derivate, insbesondere Ammonium- oder Sulfonat-funktionalisierte Polyvinylalkohole, Polyphenolen, insbesondere 1,3,5-Trihydroxybenzol, Stärke oder chemisch, mechanisch und/oder enzymatisch modifizierte Stärke, Carboxymethylcellulose und Polyvinylpyrrolidon sowie Mischungen aus den vorgenannten Verbindungen, worin die Kapselhülle umfasst oder besteht aus: eine erste Schicht aus Polyurethan und eine zweite Schicht aus Polyharnstoff, wobei sich bei der Vernetzung bei einem sauren pH-Wert eine lineare Polyharnstoff-Matrix und bei der Vernetzung bei einem alkalischen pH-Wert eine räumlich vernetzte Polyharnstoff-Matrix bildet.

Überraschend wurde festgestellt, dass die mit dem erfindungsgemäßen Verfahren hergestellten Mikrokapseln eine schichtartige Hüllstruktur bzw. mehrschichtige (Multilayer) Kapselhülle bzw. Kapselwand aufweisen, die aus einer ersten Schicht aus Polyurethan in Kernnähe der Mikrokapsel und aus einer zweiten Schicht aus Polyharnstoff am äußeren Rand der Mikrokapsel gebildet ist.

Die vorliegenden Erfindung betrifft

Isocyanat-basierte Mikrokapseln, die dadurch gekennzeichnet sind, dass die Kapselhülle umfasst oder besteht aus: eine erste Schicht aus Polyurethan und eine zweite Schicht aus Polyharnstoff.

Aufgrund der hervorragenden Stabilität und eines hervorragenden Freisetzungsvermögens der Mikrokapseln und der Möglichkeit, mit den erfindungsgemäßen Mikrokapseln ein breites Spektrum an hydrophoben Wirkstoffen zu verkapseln, sind die Isocyanat-basierten Mikrokapseln gemäß der vorliegenden Erfindung für einen breiten Anwendungsbereich zur Beduftung und Aromatisierung einsetzbar.

Die vorliegende Erfindung betrifft daher letztlich in einem weiteren Aspekt die Verwendung der erfindungsgemäßen Mikrokapseln oder von Suspensionen, die die erfindungsgemäßen Mikrokapseln umfassen, zur Herstellung von Waschmitteln, Weichspülern, Reinigungsmitteln, Scent Boostern (Duftverstärkern) in flüssiger oder fester Form, Kosmetika, Körperpflegeprodukten, Agrarprodukten oder pharmazeutischen Produkten.

Eine besonders geeignete Form des Einsatzes der erfindungsgemäßen Mikrokapseln besteht darin, sie in Form einer Suspension dem Endprodukt zuzumischen. Daher betrifft die vorliegende Erfindung auch eine Suspension der oben beschriebenen Mikrokapseln in einer Flüssigkeit, insbesondere in Wasser. Eine solche Suspension weist besonders vorteilhafte Eigenschaften auf, wenn sie so ausgestaltet ist, dass der Gewichtsanteil der Mikrokapseln bei etwa 20 bis 60 Gew.-%, insbesondere bei etwa 25 bis 50 Gew.-%, stärker bevorzugt bei etwa 30 bis 35 Gew.-% liegt.

Um ein Entmischen einer solchen Suspension zu verhindern und somit eine hohe Lagerstabilität zu erzielen, hat es sich als vorteilhaft erwiesen, dass die Suspension eine Viskosität von 12 bis 1500 mPas aufweist. Um die gewünschte Viskosität der Suspension zu erhalten wird vorzugsweise ein Verdickungsmittel eingesetzt.

Besonders geeignet sind die erfindungsgemäßen Mikrokapseln zum Einschluss von hydrophoben Duftstoffen oder Aromastoffen, die dann in verschiedenen Reinigungsprodukten eingesetzt werden können.

Die vorliegende Erfindung betrifft somit insbesondere auch die Verwendung der beschriebenen Mikrokapseln als Bestandteil von Waschmitteln, insbesondere Flüssigwaschmitteln, Weichspülern, Reinigungsmitteln, Scent Boostern (Duftverstärkern) in flüssiger oder fester Form, Kosmetika, Körperpflegeprodukten, insbesondere Duschgelen, Shampoos, Deodorants und/oder Body-Lotions.

Der Anteil an Mikrokapseln in den zuvor genannten Produkten liegt bei 0,05 bis 15 Gew.-%, bezogen auf das Gesamtgewicht des Produktes, vorzugsweise bei 0,2 bis 5 Gew.-%.

### Ausführungsbeispiele

Die Mikrokapseln der vorliegenden Erfindung und ihre vorteilhaften Eigenschaften werden anhand der nachfolgenden Beispiele näher beschrieben.

Die nachfolgend aufgeführten Stabilitätstests wurden bei 50 °C durchgeführt.

Beispiel 1: Stabilitätsdaten von Mikrokapseln, die nach dem erfindungsgemäßen Verfahren herstellt wurden, mit und ohne Katalysator (DABCO), einem ersten Vernetzer (Lysin-Hydrochlorid) bei 20 bis 30 °C und einem weiteren Vernetzer (Guanidiniumcarbonat) bei 35 bis 50 °C. Als Schutzkolloid wurde Polyvinylalkohol verwendet.

Der Stabilitätstest wurde mittels eines repräsentativen Softeners (Weichspüler) durchgeführt, in den die Mikrokapseln in einer Menge von 1 Gew.-% eingearbeitet wurde. Der Softener wurde anschließend für die u.g. Zeiträume gelagert.

Der Kapselinhalt wurde mittels GC/MS (Gaschromatographie und Massenspektrometrie) analysiert. Durch Vergleichsmessung mit einem Standard wurde der Parfümöl-Inhalt in den Kapseln bestimmt. Ein Ergebnis von beispielsweise 64 % bedeutet, dass 36 % der ursprünglich eingesetzten Menge an Parfümöl nicht mehr in der Kapsel sind.

**Tabelle 1**

| Parfümöl | 3 Tage ohne Katalysator | 10 Tage ohne Katalysator | 3 Tage mit Katalysator | 10 Tage mit Katalysator |
|---|---|---|---|---|
| Parfümöl 1 TomCap (20 % Substanz) | 64 % | 57 % | 70 % | 61% |
| Parfümöl 2 November 15 (15 % Substanz) | 71 % | 63 % | 73 % | 64% |
| Parfümöl 3 Extreme Power Safe (25 % Substanz) | 81 % | 73 % | 91 % | 85% |
| Parfümöl 4 Wonderball (30 % Substanz) | 29 % | - | 77 % | 69 % |
| Parfümöl 5 High Ball (50 % Substanz) | 10 % | - | 86 % | 78 % |
| Parfümöl 6 King of Cool (45 % Substanz) | 21 % | - | 63 % | 49 % |

| | | | | |
|---|---|---|---|---|
| Anmerkung: Substanz: Anteil an Aldehyd-, Carbonsäuren-, Ester-, primäre AlkoholKomponenten an der Parfümölzusammensetzung | | | | |

Die Parfümöle 4 bis 6 sind gekennzeichnet durch einen hohen Anteil an Komponenten mit Ester-, Aldehyd- und Carbonsäure-Funktionalität. Mit dem erfindungsgemäßen Verfahren werden auch für solche Parfümöle ausgezeichnete Stabilitätsdaten erhalten.

Beispiel 2: Stabilitätsdaten von Mikrokapseln, die nach dem erfindungsgemäßen Verfahren mit unterschiedlichen Schutzkolloiden herstellt wurden: Vernetzung mit Lysin-Hydrochlorid bei einem pH-Wert von 5,0; verkapseltes Öl: CAREN DELTA-3 NAT., CARYOPHYLLEN NAT. REKT., PHELLANDREN L, PINEN ALPHA LAEVO NAT., TERPINEN GAMMA, VERDORACINE.

**Tabelle 2**

| Schutzkolloid | 3 Tage | 10 Tage |
|---|---|---|
| Polyvinylalkohol | 83 % | 72 % |
| Kationisches Ammonium-Derivat von PVOH | 69 % | 54 % |
| Stärke | 80 % | 75 % |

Alle Mikrokapseln zeigen ausgezeichnete Stabilitätsdaten. Bei Verwendung des Ammonium-Derivats von PVOH ist die Stabilität etwas geringer, was auf die verringerte Anzahl von Hydroxylgruppen zurückzuführen ist.

Bespiel 3: Am Beispiel von Parfümöl 4 wurde eine Hüllreduktion vorgenommen. Der Gehalt an Monomer (Isocyanat) in der internen nicht-wässrigen Phase wurde reduziert.

**Tabelle 3**

| Verhältnis Monomer zu Öl | 3 Tage | 10 Tage |
|---|---|---|
| 1 : 20 | 77 % | 69 % |
| 1 : 30 | 78 % | 70 % |
| 1 : 33 | 70 % | 62 % |
| 1 : 35 | 66 % | 58 % |
| 1 : 40 | 70 % | 59 % |

| | | |
|---|---|---|
| Anmerkung: Ein Verhältnis von 1 : 20 bedeutet, dass 5 g Monomer verwendet wurden, um 100 g Parfümöl zu verkapseln | | |

Kapseln mit einem Restölgehalt von > 40 % nach 10 Tagen gelten als stabil; in diesem Fall kann die Hülle um 50 % reduziert werden.

Beispiel 4: Vergleich der Stabilität von Mikrokapseln, die nach der Verkapselungs-Technologie nach dem Stand der Technik und nach dem erfindungsgemäßen Verfahren hergestellt wurden.

### Die Mikrokapseln wurden wie folgt hergestellt:

Verkapselungs-Technologie nach dem Stand der Technik: Vernetzung: ausschließlich Polyharnstoff-Netzwerk; Katalysator: keiner; Schutzkolloid: Polyvinylalkohol; pH-Wert: 9.

Erfindungsgemäßes Verfahren: nicht-wässrigen Phase aus zwei Isocyanaten, die im Parfümöl plus Lösungsmittel gelöst wurden; wässrige Phase aus Polyvinylalkohol und DABCO; Emulgierprozess: Zugabe des im sauren pH-Wert-Bereich reagierenden Lysin-Hydrochlorids (pH-Wert: 4 bis 7); Zugabe des im alkalischen pH-Wert-Bereich reagierenden Guanidinumcarbonats (pH-Wert: 7 bis 14); pH-Wert der finalen Kapselslurry: 5 bis 8.

**Tabelle 4**

| Parfümöl | 3 Tage Technologie Stand der Technik | 10 Tage Technologie Stand der Technik | 3 Tage Technologie erfindungsgemäß | 10 Tage Technologie erfindungsgemäß |
|---|---|---|---|---|
| Parfümöl 1 TomCap (20 % Substanz) | 77 % | 70 % | 70 % | 61% |
| Parfümöl 2 November 15 (15 % Substanz) | 90 % | 85 % | 73 % | 64% |
| Parfümöl 3 Extreme Power Safe (25 % Substanz) | 94 % | 92 % | 91 % | 85% |
| Parfümöl 4 Wonderball (30 % Substanz) | 48 % | 28 % | 77 % | 69 % |
| Parfümöl 5 High Ball (50 % Substanz) | 20 % | - | 86 % | 78 % |
| Parfümöl 6 King of Cool (45 % Substanz) | 35 % | 30 % | 63% | 49% |

| | | | | |
|---|---|---|---|---|
| Anmerkung: Substanz: Anteil an Aldehyd-, Carbonsäuren-, Ester-, primäre AlkoholKomponenten an der Parfümölzusammensetzung | | | | |

Im Vergleich zur Verkapselungs-Technologie nach dem Stand der Technik ist die erfindungsgemäße Verkapselungs-Technologie universell einsetzbar, während die Technologie nach dem Stand der Technik bei Parfümölen mit einem hohen Anteil an Komponenten mit Ester-, Aldehyd- und Carbonsäure-Funktionalität einen starken Einbruch in der Stabilität aufweist. Die erfindungsgemäße Verkapselungs-Technologie weist insbesondere bei den Parfümölen 4 bis 6 Gewinne in der Stabilität auf, während die Stabilitäten bei den Parfümölen 1bis 3 geringfügig geringer ist.

### Beispiel 5: Ausbildung einer Multilayer der Kapselhülle

**Tabelle 5**

| Schutzkolloid | 3 Tage 50°C | 10 Tage 50°C |
|---|---|---|
| Stand der Technik Kapsel | 77 % | 70 % |
| Erfindungsgemäße Kapsel mit PVOH | 70 % | 61% |
| Erfindungsgemäße Kapsel mit Ammoniumderivat des PVOH | 41% | 33% |
| Erfindungsgemäße Kapsel mit Ammoniumderivat des PVOH und Glyzerin | 55% | 49% |
| Erfindungsgemäße Kapsel mit modifizierter Stärke | 76% | 67% |

Durch den Einsatz von verschiedenen Schutzkolloiden konnte nachgewiesen werden, dass sich mit dem erfindungsgemäßen Verfahren eine mehrschichtige (Multilayer) Kapselhülle bzw. Kapselwand ausbildet. Während in dem Verfahren als Schutzkolloid das Ammonium-Derivat von Polyvinylalkohol (PVOH) ohne Zusatz von weiteren Hydroxylgruppen verwendet wurde, war die Kapselstabilität deutlich geringer, als wenn in dem Verfahren zusätzlich Glycerin zugesetzt wurde, wodurch eine Erhöhung der Stabilität der Mikrokapseln beobachtet werden konnte. Durch gezieltes Weglassen von Hydroxylgruppen (siehe "erfindungsgemäße Kapsel mit Ammoniumderivat des PVOH") und gezieltes Hinzufügen von Hydroxylgruppen (siehe "erfindungsgemäße Kapsel mit Ammoniumderivat des PVOH und Glycerin") kann das Ausbilden einer Polyurethan-Schicht nachgewiesen werden, die die Stabilität massiv erhöht.

### Beispiel 6: Sensorische Evaluierung der erfindungsgemäßen Mikrokapseln

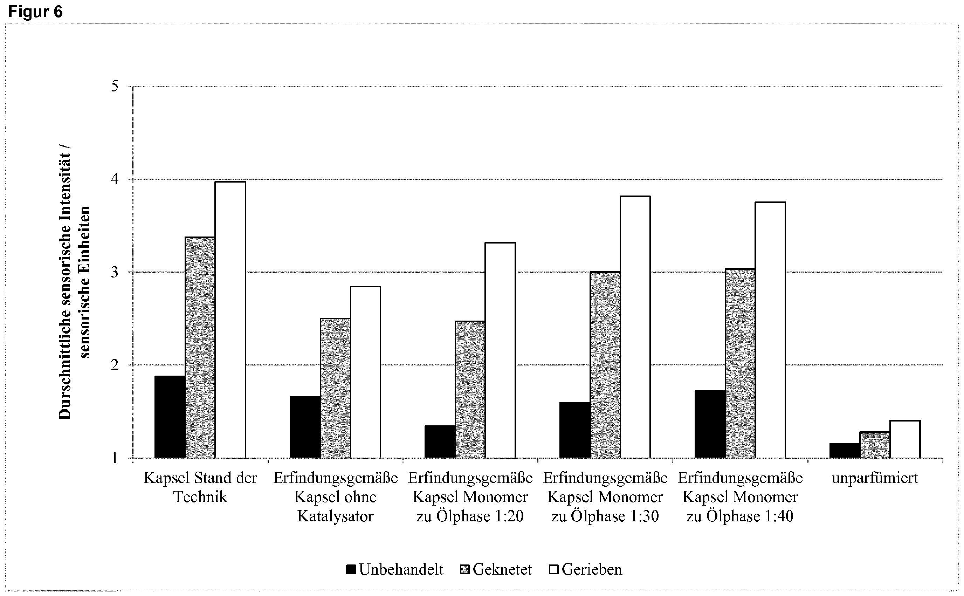

Die sensorische Evaluierung der Mikrokapseln wurde wie folgt durchgeführt: Die Mikrokapseln, wie sie in Figur 6 aufgeführt sind, wurden in einen Weichspüler eingearbeitet und dann verwaschen. Abgerochen wurde auf Mischfaser-Tüchern aus Baumwolle und Polyester.

Kapsel Stand der Technik: 10 Tage, 50 °C, 28 % Parfümöl. Erfindungsgemäße Kapsel mit reduzierter Hülle: 10 Tage, 50 °C, 59 % Parfümöl.

24 Testpersonen haben auf einer Skala von 1 (kein Geruch) bis 9 (sehr starker Geruch) die Duftintensität der Mischfaser-Tücher nach dem Waschen beurteilt.

Die erfindungsgemäßen Mikrokapseln haben faktisch den gleichen Geruch wie die Mikrokapseln aus dem Stand der Technik. Der Vorteil liegt hier an der Stabilität der erfindungsgemäßen Mikrokapseln. Diese weisen trotz Polymerreduktion von 1 : 20 auf 1 : 40, bezogen auf das Parfümöl, eine höhere Stabilität auf.

Aufgrund der oben beschriebenen vorteilhaften Eigenschaften ist auf Dauer mit den erfindungsgemäßen Mikrokapseln gegenüber den Mikrokaspeln aus dem Stand der Technik eine gleichbleibende Qualität und folglich auch sensorische Stabilität zu erwarten. Die Mikrokapseln aus dem Stand der Technik sind unmittelbar nach ihrer Herstellung genauso gut wie die erfindungsgemäßen Mikrokapseln. Über die Zeit verlieren sie jedoch schneller Öl und riechen somit weniger intensiv als die erfindungsgemäßen Mikrokapseln.

## Patentansprüche

1. Verfahren zur Herstellung von Isocyanat-basierten oder Isothiocyanatbasierten Mikrokapseln, welches die folgenden Schritte umfasst:
(a) Bereitstellen einer internen nicht-wässrigen Phase, umfassend mindestens ein Isocyanat mit zwei oder mehreren Isocyanat-Gruppen oder mindestens ein Isothiocyanat mit zwei oder mehreren Isothiocyanat-Gruppen und einen zu verkapselnden hydrophoben Wirkstoff, der eine Aldehyd-, Carbonsäure- oder Ester-Funktionalität aufweist;
(b) Bereitstellen einer externen wässrigen Phase, umfassend mindestens ein Schutzkolloid und einen Katalysator, worin das Schutzkolloid ausgewählt ist aus der Gruppe, die besteht aus Diolen, insbesondere Ethandiol, 1,2-Propandiol, 1,3-Propandiol, 1,2-Butandiol, isomeres Butandiol, 1,2-Pentandiol, 1,2-Hexandiol, 1,2-Octandiol, 1,2-Decandiol, 1,2- Dodecandiol, und Polyolen, insbesondere Triole, insbesondere Glycerin sowie dessen Ethoxylierungs- und Propoxylierungsprodukte, Trimethylolpropan sowie dessen Ethoxylierungs- und Propoxylierungsprodukte, Polyvinylalkohol (PVOH) und dessen Derivate, insbesondere Ammonium- oder Sulfonat-funktionalisierte Polyvinylalkohole, Polyphenolen, insbesondere 1,3,5-Trihydroxybenzol, Stärke oder chemisch, mechanisch und/oder enzymatisch modifizierte Stärke, Carboxymethylcellulose und Polyvinylpyrrolidon sowie Mischungen aus den vorgenannten Verbindungen;
(c) Vermischen der internen nicht-wässrigen Phase und der externen wässrigen Phase, optional in Gegenwart eines Stabilisators und/oder eines Emulgators, unter Erhalt einer Öl-in-Wasser-Emulsion;
(d) erste Vernetzung durch Zugabe eines bei einem sauren pH-Wert reagierenden Amins;
(e) weitere Vernetzung durch Zugabe eines bei einem alkalischen pH-Wert reagierenden Amins unter Erhalt von Mikrokapseln;
(f) Härten der in Schritt (e) erhaltenen Mikrokapseln; und
optional:
(g) Abtrennen der Mikrokapseln von der Reaktionslösung und gegebenenfalls Trocknen der Mikrokapseln.

2. Verfahren nach Anspruch 1, worin das Isocyanat mit zwei oder mehreren Isocyanat-Gruppen bzw. das Isothiocyanat mit zwei oder mehreren Isothiocyanat-Gruppen ausgewählt ist aus der Gruppe, die besteht aus aliphatischen, cycloaliphatischen, hydroaromatischen, aromatischen oder heterocyclischen Polyisocyanaten bzw. Polyisothiocyanaten, deren Substitutionsprodukte sowie Mischungen aus den vorgenannten Verbindungen.

3. Verfahren nach einem der vorangehenden Ansprüche 1 oder 2, worin der mindestens eine zu verkapselnde hydrophobe Wirkstoff ausgewählt ist aus der Gruppe, die besteht aus Duftstoffen, Aromastoffen, Bioziden, Insektiziden, einer Substanz aus der Gruppe der Repellentien, Lebensmittel-Additiven, kosmetischen Wirkstoffen, pharmazeutischen Wirkstoffen, Agrochemikalien, Farbstoffen. Leuchtfarben, optischen Aufhellern, Lösungsmitteln, Wachsen, Silikonölen, Schmierstoffen, sowie Mischungen aus den vorgenannten Wirkstoffen.

4. Verfahren nach einem der vorangehenden Ansprüche 1 bis 3, worin der mindestens eine zu verkapselnde hydrophobe Wirkstoff ausgewählt ist aus der Gruppe, die besteht aus Duftstoffen oder Aromastoffen, die eine Aldehyd-, Carbonsäure- oder Ester-Funktionalität aufweisen.

5. Verfahren nach einem der vorangehenden Ansprüche 1 bis 4, worin das Schutzkolloid ausgewählt ist aus der Gruppe, die besteht aus Polyolen, Polyphenolen und Stärke.

6. Verfahren nach einem der vorangehenden Ansprüche 1 bis 5, worin das Schutzkolloid in Kombination mit Stärke eingesetzt wird.

7. Verfahren nach einem der vorangehenden Ansprüche 1 bis 6, worin der Katalysator Diazobicyclo[2.2.2]octan (DABCO) ist.

8. Verfahren nach einem der vorangehenden Ansprüche 1 bis 7, worin die erste Vernetzung durch das bei einem sauren pH-Wert reagierende Amin bei einem pH-Wert von 2 bis 7 und optional bei einer Temperatur von 20 °C bis 30 °C erfolgt.

9. Verfahren nach einem der vorangehenden Ansprüche 1 bis 8, worin das sauer reagierende Amin ausgewählt ist aus der Gruppe, die besteht aus sauren Aminosäure-Hydrochloriden, insbesondere Lysin-Hydrochlorid und Ornithin-Hydrochlorid.

10. Verfahren nach einem der vorangehenden Ansprüche 1 bis 9, worin die weitere Vernetzung durch das alkalisch reagierende Amin bei einem pH-Wert von 7 bis 14 und optional bei einer Temperatur von 35 °C bis 50 °C erfolgt.

11. Verfahren nach einem der vorangehenden Ansprüche 1 bis 10, worin das alkalisch reagierende Amin ausgewählt ist aus der Gruppe, die besteht aus der Di-, Tri- und Polyamine und Guanidiniumcarbonat.

12. Verfahren nach einem der vorangehenden Ansprüche 1 bis 11, worin das Härten der Mikrokapseln in Schritt (f) bei einer Temperatur bis zu 55 bis 70 °C durchgeführt wird.

13. Isocyanat-basierte Mikrokapseln, umfassend oder bestehend aus:
- einen Kern, umfassend mindestens einen hydrophoben Wirkstoff, der eine Aldehyd-, Carbonsäure- oder Ester-Funktionalität aufweist; und
- eine Kapselhülle, umfassend ein Isocyanat mit zwei oder mehreren Isocyanat-Gruppen, das in Anwesenheit eines Schutzkolloids und eines Katalysators in einem ersten Schritt durch ein bei einem sauren pH-Wert reagierendes Amin und in einem weiteren Schritt durch ein bei einem alkalischen pH-Wert reagierendes Amin vernetzt wird, wobei das Schutzkolloid ausgewählt ist aus der Gruppe, die besteht aus Diolen, insbesondere Ethandiol, 1,2-Propandiol, 1,3-Propandiol, 1,2-Butandiol, isomeres Butandiol, 1,2-Pentandiol, 1,2-Hexandiol, 1,2-Octandiol, 1,2-Decandiol, 1,2- Dodecandiol, und Polyolen, insbesondere Triole, insbesondere Glycerin sowie dessen Ethoxylierungs- und Propoxylierungsprodukte, Trimethylolpropan sowie dessen Ethoxylierungs- und Propoxylierungsprodukte, Polyvinylalkohol (PVOH) und dessen Derivate, insbesondere Ammonium- oder Sulfonat-funktionalisierte Polyvinylalkohole, Polyphenolen, insbesondere 1,3,5-Trihydroxybenzol, Stärke oder chemisch, mechanisch und/oder enzymatisch modifizierte Stärke, Carboxymethylcellulose und Polyvinylpyrrolidon sowie Mischungen aus den vorgenannten Verbindungen,
worin die Kapselhülle umfasst oder besteht aus: eine erste Schicht aus Polyurethan und eine zweite Schicht aus Polyharnstoff, wobei sich bei der Vernetzung bei einem sauren pH-Wert eine lineare Polyharnstoff-Matrix und bei der Vernetzung bei einem alkalischen pH-Wert eine räumlich vernetzte Polyharnstoff-Matrix bildet.

14. Verwendung der Mikrokapseln nach Anspruch 13 oder einer Suspension aus Mikrokapseln nach Anspruch 13 zur Herstellung von Waschmitteln, Weichspülern, Reinigungsmitteln, Duftverstärkern in flüssiger oder fester Form, Kosmetika, Körperpflegeprodukten, Agrarprodukten oder pharmazeutischen Produkten.

## Claims

1. Process for preparing isocyanate-based or isothiocyanate-based microcapsules, which comprises the following steps:
(a) providing an internal non-aqueous phase, comprising at least one isocyanate having two or more isocyanate groups or at least one isothiocyanate having two or more isothiocyanate groups, and comprising a hydrophobic active ingredient to be encapsulated having an aldehyde, carboxylic acid or ester functionality;
(b) providing an external aqueous phase, comprising at least one protective colloid and a catalyst, wherein the protective colloid is selected from the group consisting of diols, in particular ethanediol, 1,2-propanediol, 1,3-propanediol, 1,2-butanediol, isomeric butanediol, 1,2-pentanediol, 1,2-hexanediol, 1,2-octanediol, 1,2-decanediol, 1,2-dodecanediol, and polyols, in particular triols, in particular glycerol and ethoxylation and propoxylation products thereof, trimethylolpropane and ethoxylation and propoxylation products thereof, polyvinyl alcohol (PVOH) and derivatives thereof, in particular ammonium- or sulfonate-functionalized polyvinyl alcohols, polyphenols, in particular 1,3,5-trihydroxybenzene, starch or chemically, mechanically and/or enzymatically modified starch, carboxymethylcellulose and polyvinylpyrrolidone, and mixtures of the aforementioned compounds;
(c) mixing the internal non-aqueous phase and the external aqueous phase, optionally in the presence of a stabilizer and/or an emulsifier, to obtain an oil-in-water emulsion;
(d) first crosslinking by adding an amine that reacts at an acidic pH;
(e) further crosslinking by adding an amine that reacts at an alkaline pH, so as to obtain microcapsules;
(f) curing the microcapsules obtained in step (e); and
optionally:
(g) separating the microcapsules from the reaction solution and, if necessary, drying the microcapsules.

2. Process according to claim 1, wherein the isocyanate having two or more isocyanate groups or the isothiocyanate having two or more isothiocyanate groups is selected from the group consisting of aliphatic, cycloaliphatic, hydroaromatic, aromatic or heterocyclic polyisocyanates or polyisothiocyanates, substitution products thereof, and mixtures of the aforementioned compounds.

3. Process according to any of the preceding claims 1 or 2, wherein the at least one hydrophobic active ingredient to be encapsulated is selected from the group consisting of fragrances, flavorings, biocides, insecticides, a substance from the group of repellents, food additives, cosmetic active ingredients, pharmaceutical active ingredients, agrochemicals, dyes, fluorescent dyes, optical brighteners, solvents, waxes, silicone oils, lubricants, and mixtures of the aforementioned active ingredients.

4. Process according to any of the preceding claims 1 to 3, wherein the at least one hydrophobic active ingredient to be encapsulated is selected from the group consisting of fragrances or flavorings having an aldehyde, carboxylic acid or ester functionality.

5. Process according to any of the preceding claims 1 to 4, wherein the protective colloid is selected from the group consisting of polyols, polyphenols and starch.

6. Process according to any of the preceding claims 1 to 5, wherein the protective colloid is used in combination with starch.

7. Process according to any of the preceding claims 1 to 6, wherein the catalyst is diazobicyclo[2.2.2]octane (DABCO).

8. Process according to any of the preceding claims 1 to 7, wherein the first crosslinking by the amine that reacts at an acidic pH takes place at a pH of 2 to 7 and optionally at a temperature of 20°C to 30°C.

9. Process according to any of the preceding claims 1 to 8, wherein the acid-reacting amine is selected from the group consisting of acidic amino acid hydrochlorides, in particular lysine hydrochloride and ornithine hydrochloride.

10. Process according to any of the preceding claims 1 to 9, wherein the further crosslinking by the alkaline-reacting amine takes place at a pH of 7 to 14 and optionally at a temperature of 35°C to 50°C.

11. Process according to any of the preceding claims 1 to 10, wherein the alkaline-reacting amine is selected from the group consisting of di-, tri- and polyamines and guanidinium carbonate.

12. Process according to any of the preceding claims 1 to 11, wherein the curing of the microcapsules in step (f) is carried out at a temperature of up to 55 to 70°C.

13. Isocyanate-based microcapsules, comprising or consisting of:
- a core, comprising at least one hydrophobic active ingredient having an aldehyde, carboxylic acid or ester functionality; and
- a capsule shell, comprising an isocyanate having two or more isocyanate groups, which, in the presence of a protective colloid and a catalyst, is crosslinked in a first step by an amine that reacts at an acidic pH and in a further step by an amine that reacts at an alkaline pH, wherein the protective colloid is selected from the group consisting of diols, in particular ethanediol, 1,2-propanediol, 1,3-propanediol, 1,2-butanediol, isomeric butanediol, 1,2-pentanediol, 1,2-hexanediol, 1,2-octanediol, 1,2-decanediol, 1,2-dodecanediol, and polyols, in particular triols, in particular glycerol and ethoxylation and propoxylation products thereof, trimethylolpropane and ethoxylation and propoxylation products thereof, polyvinyl alcohol (PVOH) and derivatives thereof, in particular ammonium- or sulfonate-functionalized polyvinyl alcohols, polyphenols, in particular 1,3,5-trihydroxybenzene, starch or chemically, mechanically and/or enzymatically modified starch, carboxymethylcellulose and polyvinylpyrrolidone, and mixtures of the aforementioned compounds, wherein the capsule shell comprises or consists of: a first layer of polyurethane and a second layer of polyurea, wherein a linear polyurea matrix is formed during the crosslinking at an acidic pH and a spatially crosslinked polyurea matrix is formed during the crosslinking at an alkaline pH.

14. Use of the microcapsules according to claim 13 or of a suspension of microcapsules according to claim 13 to produce detergents, fabric softeners, cleaning agents, scent enhancers in liquid or solid form, cosmetics, personal care products, agricultural products or pharmaceutical products.

## Revendications

1. Procédé de production de microcapsules à base d'isocyanate ou à base d'isothiocyanate, comprenant les étapes suivantes :
(a) préparation d'une phase non aqueuse interne comprenant au moins un isocyanate ayant deux ou plusieurs groupes isocyanates ou au moins un isothiocyanate ayant deux ou plusieurs groupes isothiocyanates et un agent actif hydrophobe à encapsuler présentant une fonctionnalité aldéhyde, acide carboxylique ou ester ;
(b) préparation d'une phase aqueuse externe comprenant au moins un colloïde protecteur et un catalyseur, où le colloïde protecteur est choisi dans le groupe constitué de diols, en particulier l'éthanediol, le 1,2-propanediol, le 1,3-propanediol, le 1,2-butanediol, le butanediol isomérique, le 1,2-pentanediol, le 1,2-hexanediol, le 1,2-octanediol, le 1,2-décanediol, le 1,2-dodécanediol, et de polyols, en particulier des triols, notamment le glycérol ainsi que ses produits d'éthoxylation et de propoxylation, le triméthylolpropane ainsi que ses produits d'éthoxylation et de propoxylation, l'alcool polyvinylique (PVOH) et ses dérivés, en particulier les alcools polyvinyliques fonctionnalisés par des ammoniums ou des sulfonates, les polyphénols, en particulier le 1,3,5-trihydroxybenzène, l'amidon ou l'amidon modifié chimiquement, mécaniquement et/ou enzymatiquement, la carboxyméthylcellulose et la polyvinylpyrrolidone ainsi que des mélanges des composés précités ;
(c) mélange de la phase non aqueuse interne et de la phase aqueuse externe, facultativement en présence d'un stabilisant et/ou d'un émulsifiant, pour obtenir une émulsion huile-dans-eau ;
(d) première réticulation par ajout d'une amine réagissant à un pH acide ;
(e) réticulation supplémentaire par ajout d'une amine réagissant à un pH alcalin, avec obtention de microcapsules ;
(f) durcissement des microcapsules obtenues lors de l'étape (e) ; et
facultativement :
(g) séparation des microcapsules de la solution réactionnelle et, le cas échéant, séchage des microcapsules.

2. Procédé selon la revendication 1, où l'isocyanate ayant deux ou plusieurs groupes isocyanates ou l'isothiocyanate ayant deux ou plusieurs groupes isothiocyanates est choisi dans le groupe constitué de polyisocyanates ou de polyisothiocyanates aliphatiques, cycloaliphatiques, hydroaromatiques, aromatiques ou hétérocycliques, leurs produits de substitution ainsi que des mélanges des composés précités.

3. Procédé selon la revendication 1 ou la revendication 2, où ledit au moins un agent hydrophobe à encapsuler est choisi dans le groupe constitué de parfums, de substances aromatiques, de biocides, d'insecticides, d'une substance du groupe des répulsifs, d'additifs alimentaires, d'agents cosmétiques, d'agents pharmaceutiques, de produits agrochimiques, de colorants, de peintures lumineuses, d'azurants optiques, de solvants, de cires, d'huiles de silicone, de lubrifiants, ainsi que de mélanges des substances actives précitées.

4. Procédé selon l'une des revendications 1 à 3, où ledit au moins un principe actif hydrophobe à encapsuler est choisi dans le groupe constitué de parfums ou d'arômes présentant une fonctionnalité aldéhyde, acide carboxylique ou ester.

5. Procédé selon l'une des revendications 1 à 4, où le colloïde protecteur est choisi dans le groupe constitué de polyols, de polyphénols et d'amidon.

6. Procédé selon l'une des revendications 1 à 5, où le colloïde protecteur est utilisé en combinaison avec l'amidon.

7. Procédé selon l'une des revendications 1 à 6, où le catalyseur est le diazobicyclo[2.2.2]octane (DABCO).

8. Procédé selon l'une des revendications 1 à 7, où la première réticulation est effectuée par l'amine réagissant à un pH acide, à un pH de 2 à 7 et facultativement à une température de 20 °C à 30 °C.

9. Procédé selon l'une des revendications 1 à 8, où l'amine à réaction acide est choisie dans le groupe constitué de chlorhydrates d'acides aminés, en particulier le chlorhydrate de lysine et le chlorhydrate d'ornithine.

10. Procédé selon l'une des revendications 1 à 9, où la réticulation supplémentaire est effectuée par l'amine à réaction alcaline à un pH de 7 à 14 et facultativement à une température de 35 °C à 50 °C.

11. Procédé selon l'une des revendications 1 à 10, où l'amine à réaction alcaline est choisie dans le groupe constitué de di-, tri- et polyamines et de carbonate de guanidinium.

12. Procédé selon l'une des revendications 1 à 11, où le durcissement des microcapsules lors de l'étape (f) est effectué à une température de 55 à 70 °C.

13. Microcapsules à base d'isocyanate, comprenant ou consistant en :
- un noyau comprenant au moins un agent actif hydrophobe ayant une fonctionnalité aldéhyde, acide carboxylique ou ester ; et
- une enveloppe de capsule comprenant un isocyanate ayant deux ou plusieurs groupes isocyanates, réticulé en présence d'un colloïde protecteur et d'un catalyseur, lors d'une première étape par une amine réagissant à un pH acide et lors d'une autre étape par une amine réagissant à un pH alcalin, le colloïde protecteur étant choisi dans le groupe constitué de diols, en particulier l'éthanediol, le 1,2-propanediol, le 1,3-propanediol, le 1,2-butanediol, le butanediol isomérique, le 1,2-pentanediol, le 1,2-hexanediol, le 1,2-octanediol, le 1,2-décanediol, 1,2-dodécanediol, et de polyols, en particulier des triols, notamment le glycérol et ses produits d'éthoxylation et de propoxylation, le triméthylolpropane et ses produits d'éthoxylation et de propoxylation, l'alcool polyvinylique (PVOH) et ses dérivés, en particulier les alcools polyvinyliques fonctionnalisés par des ammoniums ou des sulfonates, les polyphénols, en particulier le 1,3,5-trihydroxybenzène, l'amidon ou l'amidon modifié chimiquement, mécaniquement et/ou enzymatiquement, la carboxyméthylcellulose et la polyvinylpyrrolidone ainsi que les mélanges des composés précités,
où l'enveloppe de capsule comprend ou consiste en : une première couche de polyuréthane et une deuxième couche de polyurée, une matrice linéaire de polyurée se formant lors de la réticulation à un pH acide et une matrice spatialement réticulée de polyurée se formant lors de la réticulation à un pH alcalin.

14. Utilisation des microcapsules selon la revendication 13 ou d'une suspension de microcapsules selon la revendication 13 pour la fabrication de détergents, d'adoucissants, de produits de nettoyage, de renforçateurs de parfum sous forme liquide ou solide, de produits cosmétiques, de produits de soins corporels, de produits agricoles ou de produits pharmaceutiques.
